# EUROPEAN PATENT APPLICATION

(11) **EP 4 095 240 A1**
(43) Date of publication of application: **30.11.2022**
(21) Application number: 21744251.6
(22) Date of filing: 22.01.2021
(51) Int. Cl.: C12N 5/0789

(54) **SERUM-FREE MEDIUM AND CULTURING METHOD SUITED FOR CULTURING BLOOD CELLS SUCH AS HUMAN HEMATOPOIETIC STEM CELLS**

(30) Priority: 24.01.2020 JP 2020010390
(71) Applicant: The University of Tokyo, Bunkyo-ku, Tokyo 113-8654 (JP)
(72) Inventor: YAMAZAKI, Satoshi, Tokyo 113-8654 (JP); WATANABE, Motoo, Tokyo 113-8654 (JP)
(74) Representative: Gulde & Partner
(86) International application number: PCT/JP2021/002252
(87) International publication number: WO 2021/149799

(57) **Abstract**

The present invention discloses a serum-free medium and a culture method suitable for culturing human cells. The present invention provides a method of culturing human cells, the method comprising bringing the human cells into contact with polyalkylene glycol modified with a copolymer containing a polyvinylcaprolactam block and a polyvinylacetate block.

## Description

### Technical Field

The present invention discloses a composition of serum-free (especially albumin-free) medium and a culture method suitable for culturing blood cells such as hematopoietic stem cells. The present invention provides a method of culturing blood cells such as human hematopoietic stem cells. This method can include bringing blood cells, such as human hematopoietic stem cells, into contact with polyethylene glycol modified with a moiety having a polyvinylcaprolactam block and a polyvinylacetate block linked.

### Background Art

To culture hematopoietic stem cells, the use of a chemically defined medium for their proliferation has been studied. For mouse hematopoietic stem cells, it has been shown that they can be cultured in a chemically defined medium (Non Patent Literature 1).

### Citation List

### Non Patent Literature

Non Patent Literature 1: Wilkinson et al., Nature, 571: 117-121, 2019

### Summary of Invention

The present invention provides a culture method and a composition of a medium (e.g., a serum-free medium (e.g., an albumin-free, serum-free medium or cytokine-free, serum-free medium, such as an albumin-free and cytokine-free, serum-free medium)) suitable for culturing blood cells such as human hematopoietic stem cells.

The present inventors have found and reported that mouse hematopoietic stem cells (sometimes referred to as KSL cells due to their isolation method) can be grown in large numbers over a long period of time by adding polyvinyl alcohol (PVA) to an albumin-free, serum-free medium (Wilkinson et al., Nature, 571: 117-121, 2019). However, no method has been established for expansion of human hematopoietic stem cells in an albumin-free, serum-free medium. The present inventors have recently found that human hematopoietic stem cells exhibit marked proliferation in the presence of polyethylene glycol modified with a copolymer containing a polyvinylcaprolactam block and a polyvinylacetate block, even in an albumin-free, serum-free medium, while maintaining their stem cell characteristics. In addition, it has also been found that the proliferation of hematopoietic stem cells can be maintained even under cytokine-free conditions at that time. In addition to hematopoietic stem cells, it has further been found that various blood cells, including erythroblasts, megakaryocytes, and leukocytes (e.g., T cells, B cells, macrophages, neutrophils), can proliferate under the same conditions.

The invention can provide the following items of the invention.
[1] A method of culturing human hematopoietic stem cells or human blood cells, comprising:
   culturing the human hematopoietic stem cells or human blood cells in a culture medium, wherein
   the culture medium is a polyvinyl alcohol-containing albumin-free medium (especially a serum albumin-free medium), and comprises:
      (1) a phosphatidylinositol 3-kinase (PI3K) activator and at least one compound selected from the group consisting of thrombopoietin (TPO) and TPO receptor agonists;
      (2) a TPO receptor agonist and at least one compound selected from the group consisting of stem cell factor (SCF) and PI3K activators; or
      (3) a PI3K activator and a TPO receptor agonist, so that
   the culturing allows the number of the human hematopoietic stem cells or human blood cells to increase or be maintained.
[2] The method according to item [1], wherein the increased number of the human hematopoietic stem cells or human blood cells is obtained.
[3] The method according to item [1] or [2], wherein the culture medium comprises a PI3K activator and is free of stem cell factor (SCF).
[4] The method according to any one of items [1] to [3], wherein the culture medium comprises a PI3K activator and a TPO receptor agonist.
[5] The method according to item [4], wherein the culture medium comprises neither SCF nor TPO.
[5A] The method according to item [4] or [5], wherein the culture medium is a cytokine-free medium.
[6] The method according to any one of items [1] to [5], wherein the culture medium further comprises 4-N-[2-benzyl-7-(2-methyltetrazol-5-yl)-9H-pyrimid[4,5-b]indol-4-yl]cyclohexane-1,4-diamine (UM171).
[7] The method according to item [6], wherein the culturing has a period of 7 days or longer.
[8] An albumin-free composition comprising
   human hematopoietic stem cells or human blood cells, polyvinyl alcohol, and
   (1) a PI3K activator and at least one compound selected from the group consisting of TPO and TPO receptor agonists;
   (2) a TPO receptor agonist and at least one compound selected from the group consisting of stem cell factor (SCF) and PI3K activators; or
   (3) a PI3K activator and a TPO receptor agonist.
[9] The composition according to item [8], wherein the composition comprises a PI3K activator and a TPO receptor agonist.
[10] The composition according to item [8] or [9], further comprising 4-N-[2-benzyl-7-(2-methyltetrazol-5 -yl)-9H-pyrimid[4,5-b]indol-4-yl]cyclohexane-1,4-diamine (UM171).
[11] Human hematopoietic stem cells or human blood cells obtained by the method according to any one of items [1] to [7].
[12] A polyvinyl alcohol-containing, albumin-free culture medium for human hematopoietic stem cells or human blood cells, comprising:
   (1) a PI3K activator and at least one compound selected from the group consisting of TPO and TPO receptor agonists;
   (2) a TPO receptor agonist and at least one compound selected from the group consisting of stem cell factor (SCF) and PI3K activators; or
   (3) a PI3K activator and a TPO receptor agonist.

{1} A method of culturing or producing human hematopoietic stem cells or human blood cells, comprising:
   culturing the human hematopoietic stem cells or human blood cells in a culture medium, wherein
   the culture medium comprises polyvinyl alcohol and
      (1) a phosphatidylinositol 3-kinase (PI3K) activator and at least one compound selected from the group consisting of thrombopoietin (TPO) and TPO receptor agonists;
      (2) a TPO receptor agonist and at least one compound selected from the group consisting of stem cell factor (SCF) and PI3K activators; or
      (3) a PI3K activator and a TPO receptor agonist, so that
   the culturing allows the number of the human hematopoietic stem cells or human blood cells to increase or be maintained.
{2} The method according to item {1}, wherein the culture medium is a serum-free medium.
{3} The method according to item {1}, wherein the culture medium is a chemically defined medium.
{4} The method according to any one of items {1} to {3}, wherein the culture medium is substantially free of albumin.
{5} The method according to any one of items {1} to {4}, wherein the increased number of the human hematopoietic stem cells or human blood cells is obtained.
{6} The method according to any one of items {1} to {5}, wherein the culture medium comprises a PI3K activator and is free of stem cell factor (SCF).
{7} The method according to any one of items {1} to {6}, wherein the culture medium comprises a PI3K activator and a TPO receptor agonist.
{8} The method according to item {7}, wherein the culture medium comprises neither SCF nor TPO.
{8A} The method according to item {7} or {8}, wherein the culture medium is a cytokine-free medium.
{9} The method according to any one of items {1} to {8}, wherein the culture medium further comprises 4-N-{2-benzyl-7-(2-methyltetrazol-5-yl)-9H-pyrimid{4,5-b}indol-4-yl}cyclohexane-1,4-diamine (UM171).
{10} The method according to item {9}, wherein the culturing has a period of 7 days or longer.
{11} A composition comprising human hematopoietic stem cells or human blood cells, polyvinyl alcohol as an alternative for albumin, and
   (1) a PI3K activator and at least one compound selected from the group consisting of TPO and TPO receptor agonists;
   (2) a TPO receptor agonist and at least one compound selected from the group consisting of stem cell factor (SCF) and PI3K activators; or
   (3) a PI3K activator and a TPO receptor agonist.
{12} The composition according to item {11}, wherein the composition comprises a PI3K activator and a TPO receptor agonist.
{13} The composition according to item {11} or {12}, further comprising 4-N-{2-benzyl-7-(2-methyltetrazol-5-yl)-9H-pyrimid{4,5-b}indol-4-yl}cyclohexane-1,4-diamine (UM171).
{14} Human hematopoietic stem cells or human blood cells obtained by the method according to any one of items {1} to {10}.
{15} An albumin alternative polyvinyl alcohol-containing culture medium for human hematopoietic stem cells or human blood cells, comprising:
   (1) a PI3K activator and at least one compound selected from the group consisting of TPO and TPO receptor agonists;
   (2) a TPO receptor agonist and at least one compound selected from the group consisting of stem cell factor (SCF) and PI3K activators; or
   (3) a PI3K activator and a TPO receptor agonist.
{16} The culture medium according to item {15}, which is a serum-free medium.
{17} The culture medium according to item {15}, which is a chemically defined medium.
{18} The culture medium according to any one of items {15} to {17}, which is free of albumin.
{19} The method according to any one of {1} to {8}, wherein the human cells are human hematopoietic stem cells.
{20} The composition according to any one of {11} to {13}, wherein the human cells are human hematopoietic stem cells.
{21} The method according to any one of {1} to {8}, wherein the human cells are human blood cells.
{22} The composition according to any one of {11} to {13}, wherein the human cells are human blood cells.
{23} A culture medium comprising human cells obtained by the method according to any one of items {1} to {8}, {19}, and {21}.
{24} The culture medium according to item {23}, which is an albumin-free, cytokine-free, serum-free medium.

The invention can also provide the following items of the invention.
[1B] A method of culturing human cells, comprising
culturing human cells in a culture medium, wherein
the culture medium comprises polyalkylene glycol modified with a copolymer containing a polyvinylcaprolactam block and a polyvinylacetate block.
[2B] The method according to item [1B], wherein the increased number of the human cells is obtained.
[3B] The method according to item [1B] or [2B], wherein the human cells are human hematopoietic stem cells or human blood cells.
[4B] A culture medium composition for culturing human cells, comprising polyalkylene glycol modified with a copolymer containing a polyvinylcaprolactam block and a polyvinylacetate block.
[5B] A composition comprising human cells and polyalkylene glycol modified with a copolymer containing a polyvinylcaprolactam block and a polyvinylacetate block.
[6B] The composition according to item [4B] or [5B], wherein the human cells are human hematopoietic stem cells or human blood cells.
[7B] Human cells obtained by the method according to any one of items [1B] to [3B].
[8B] The method according to item [3B], wherein the human cells are human hematopoietic stem cells.
[9B] The composition according to item [6B], wherein the human cells are human hematopoietic stem cells.
[10B] The method according to item [3B], wherein the human cells are human blood cells.
[11B] The composition according to item [6B], wherein the human cells are human blood cells.
[12B] A culture medium containing human cells obtained by the method according to any one of items [1B] to [3B].
[13B] The culture medium according to item [12B], which is an albumin-free, cytokine-free, serum-free medium.
[14B] The method according to any one of items [1B] to [3B], [8B], and [10B], the composition according to any one of items [4B] to[6B], [9B], and [11B], or the culture medium according to item [12] or [13], wherein the polyalkylene glycol modified with a copolymer containing a polyvinylcaprolactam block and a polyvinylacetate block is polyethylene glycol modified with a copolymer containing a polyvinylcaprolactam block and a polyvinylacetate block.

[1C] A method of culturing human cells, comprising
culturing human cells in a culture medium, wherein
the culture medium is a serum albumin-free medium and comprises an additive, the additive being selected from the group consisting of polyvinyl alcohol and modified polyalkylene glycol, and further comprises
   (1) a phosphatidylinositol 3-kinase (PI3K) activator and at least one compound selected from the group consisting of thrombopoietin (TPO) and TPO receptor agonists;
   (2) a TPO receptor agonist and at least one compound selected from the group consisting of stem cell factor (SCF) and PI3K activators; or
   (3) a PI3K activator and a TPO receptor agonist, so that
the culturing allows the number of human hematopoietic stem cells or human blood cells to increase or be maintained.
[2C] The method according to item [1C], wherein the human cells are cells selected from the group consisting of human hematopoietic stem cells and human blood cells.
[3C] The method according to item [1C], wherein the human cells are blood cells other than hematopoietic stem cells.
[4C] The method according to item [3C], wherein the additive is polyvinyl alcohol.
[5C] The method according to item [1C] or [2C], wherein the additive is modified polyalkylene glycol.
[6C] The method according to item [5C], wherein the modified polyalkylene glycol is polyalkylene glycol modified with a copolymer containing a polyvinylcaprolactam block and a polyvinylacetate block.
[7C] The method according to item [6C], wherein the modified polyalkylene glycol is polyethylene glycol modified with a copolymer containing a polyvinylcaprolactam block and a polyvinylacetate block.

### Brief Description of Drawings

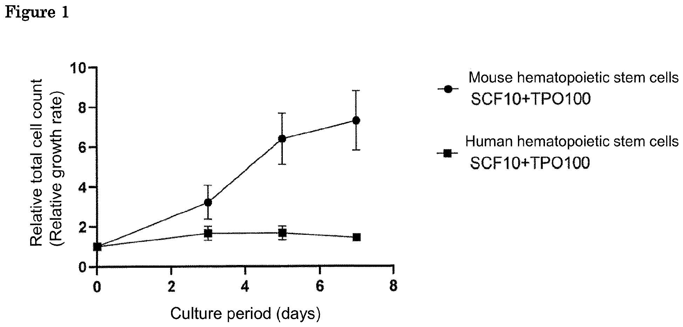

[Figure 1] FIG. 1 shows the results of culturing, in the presence of 10 ng/mL mouse or human stem cell factor (SCF) and 100ng /mL of thrombopoietin (TPO), mouse hematopoietic stem cells (mouse KSL cells) or human hematopoietic stem cells (CD34⁺CD38⁻ cells) in an albumin-free, serum-free medium containing polyvinyl alcohol (PVA).

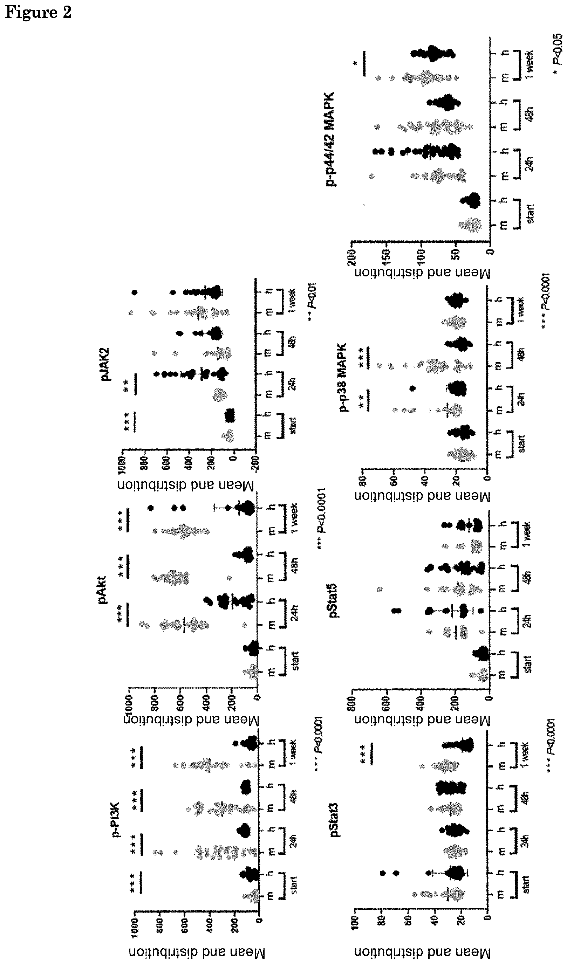

[Figure 2] FIG. 2 is graphs indicating the degree of phosphorylation of each signaling factor downstream of SCF and TPO in mouse or human hematopoietic stem cells in the presence of 10 ng/mL stem cell factor (SCF) and 100 ng/mL thrombopoietin (TPO), in the absence of albumin, and in the presence of PVA. The sign "m" represents mouse hematopoietic stem cells, and the "h" represents human hematopoietic stem cells.

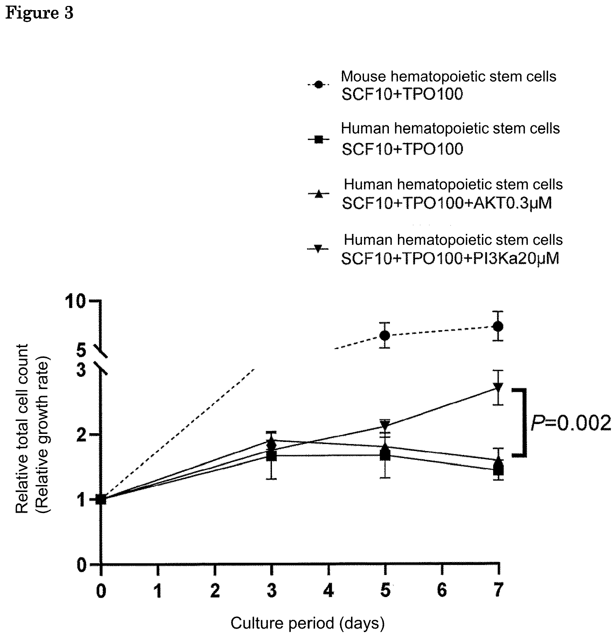

[Figure 3] FIG. 3 shows the results of culturing, in the absence of albumin and in the presence of PVA, human hematopoietic stem cells in a medium containing 10 ng/mL human stem cell factor (SCF) and 100 ng/mL human thrombopoietin in the presence of an AKT Activator II (AKTa) or PI3K activator (PI3Ka).

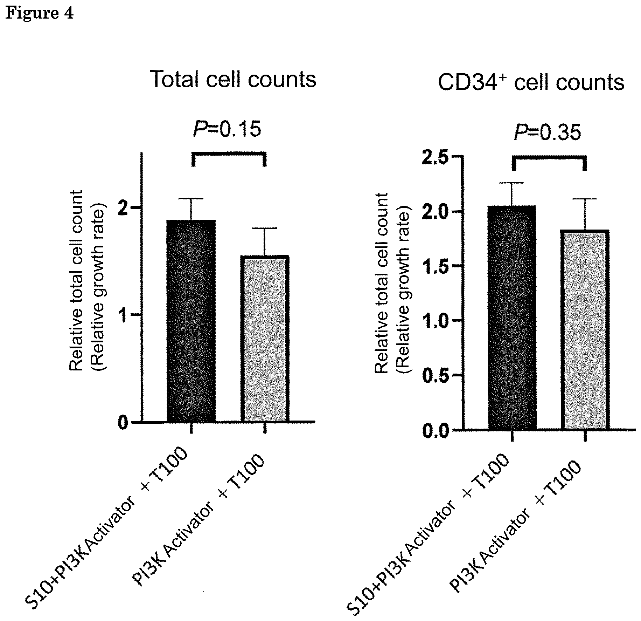

[Figure 4] FIG. 4 shows the growth rate of total cells or CD34⁺ cells at day 7 after human hematopoietic stem cells were cultured in the absence of albumin and in the presence of PVA and 100 ng/mL human thrombopoietin (TPO). In FIG. 4, SCF-containing conditions and SCF-free conditions are compared. FIG. 4 indicates that no statistically significant difference in the number of total cells or CD34⁺ cells at culture day 7 was found between the two conditions.

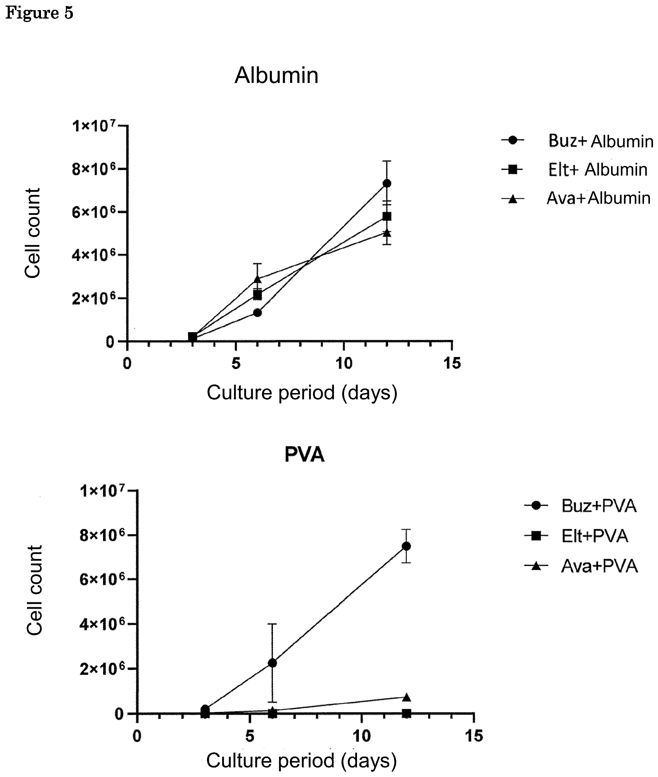

[Figure 5] FIG. 5 shows the time course of the number of cultured human hematopoietic stem cells in the presence of albumin or PVA while TPO was replaced by each TPO receptor agonist. The sign "Buty" represents butyzamide, the "Elt" represents eltrombopag, and the "Ava" represents avatrombopag.

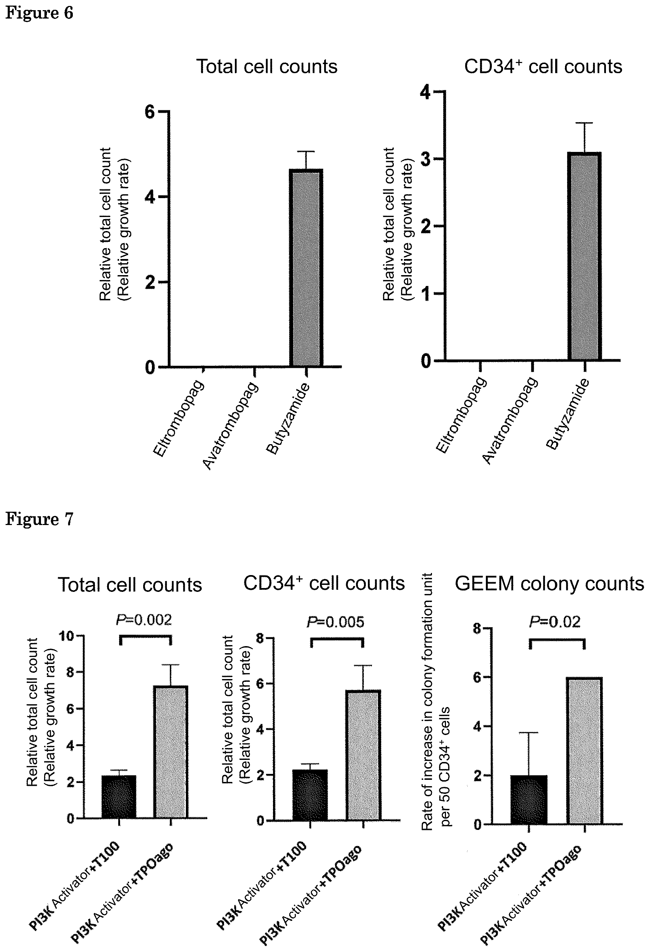

[Figure 6] FIG. 6 shows the growth rate of total cells or CD34⁺ cells at day 7 when human hematopoietic stem cells were cultured in the presence of albumin or PVA while TPO was replaced by each TPO receptor agonist.
[Figure 7] FIG. 7 indicates the number of total cells, the number of CD34⁺ cells, or the number of GEmM colonies at day 7 when human hematopoietic stem cells were cultured in a culture medium containing a PI3K activator and TPO or butyzamide (Buty) in the absence of albumin and in the presence of PVA. The types of colonies were identified under a microscope after each colony was picked under a microscope, a cytospin sample was prepared, and the sample was subjected to Giemsa staining. G denotes "granulocytes", E denotes "erythroblasts", m denotes "macrophages", and M denotes "megakaryocytes".

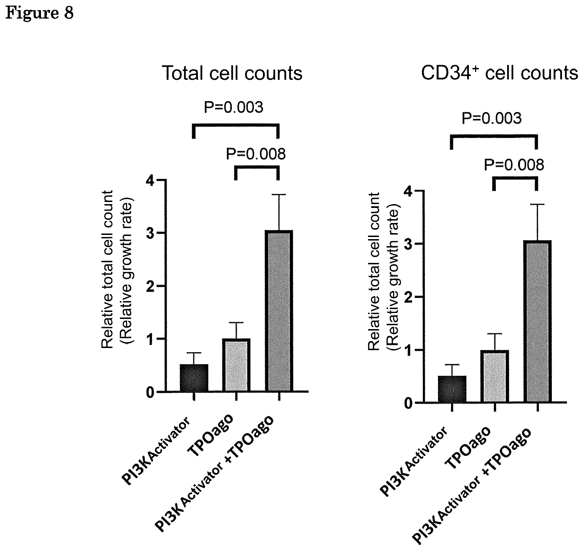

[Figure 8] FIG. 8 shows the growth rate of total cells or CD34⁺ cells at day 7 when human hematopoietic stem cells were cultured in the absence of albumin and in the presence of PVA in a culture medium without SCF or TPO but containing a PI3K activator, a TPO receptor agonist, or a combination of them.

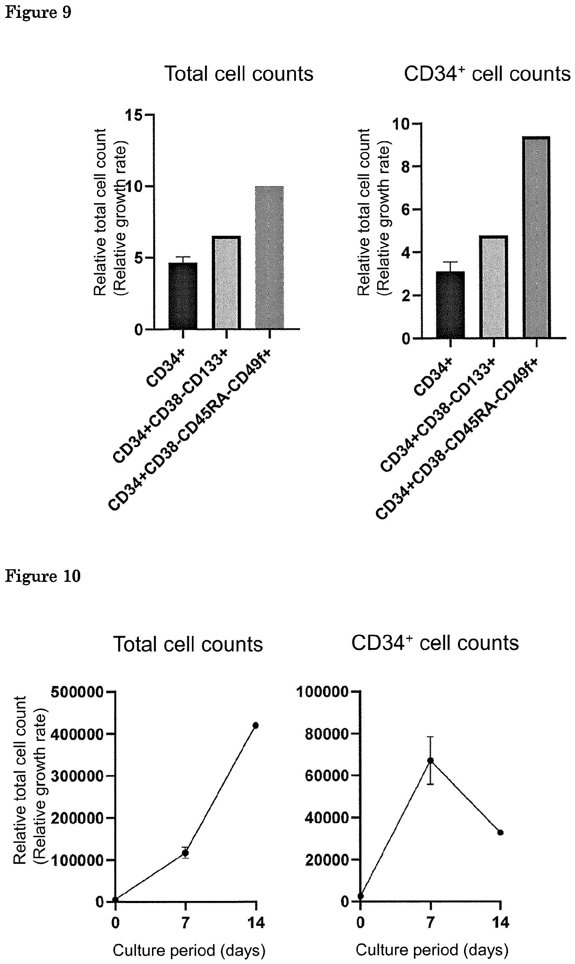

[Figure 9] FIG. 9 shows the growth rate of each cell population in a culture obtained at day 7 when human hematopoietic stem cells were cultured in the absence of albumin and in the presence of PVA in a culture medium without SCF or TPO but containing a PI3K activator and a TPO receptor agonist.
[Figure 10] FIG. 10 shows the time course of the number of total cells or the number of CD34⁺ cells at days 7 and 14 when human hematopoietic stem cells were cultured in the absence of albumin and in the presence of PVA in a culture medium without SCF or TPO but containing a PI3K activator and a TPO receptor agonist.

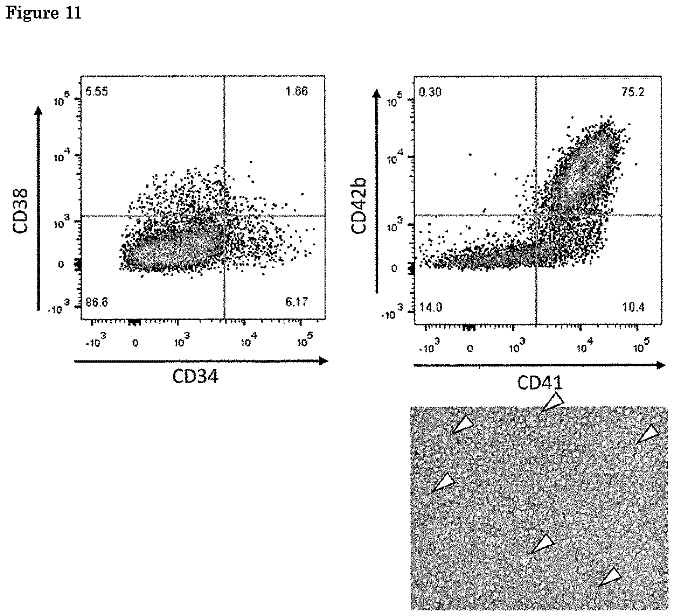

[Figure 11] FIG. 11 shows the results of gated cells obtained at day 14 when human hematopoietic stem cells were cultured in the absence of albumin and in the presence of PVA in a culture medium without SCF or TPO but containing a PI3K activator and a TPO receptor agonist. The left panel shows the results of flow cytometry using CD34 and CD38 as markers, and the right panel shows the results of flow cytometry using CD41a and CD42b as markers. A photograph of FIG. 11 is an optical micrograph of the obtained culture.

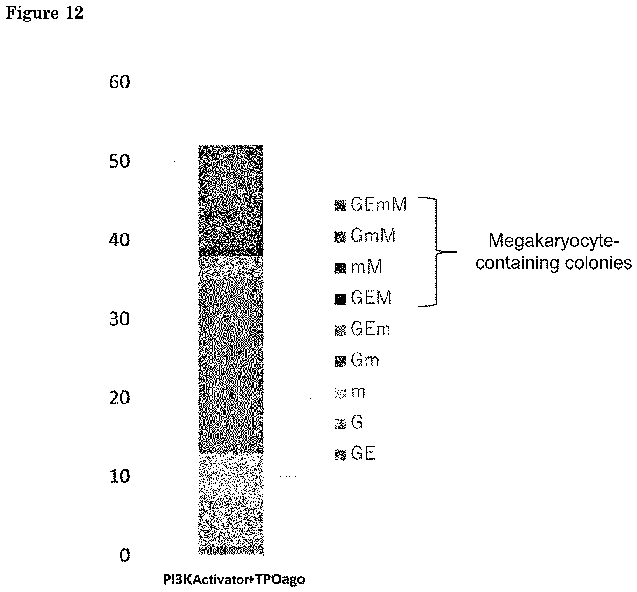

[Figure 12] FIG. 12 shows the results of colony assay using cells obtained at day 14 when human hematopoietic stem cells were cultured in the absence of albumin and in the presence of PVA in a culture medium without SCF or TPO but containing a PI3K activator and a TPO receptor agonist. The types of colonies were identified under a microscope after each colony was picked under a microscope, a cytospin sample was prepared, and the sample was subjected to Giemsa staining. G denotes "granulocytes"-, E denotes "erythroblasts"-, m denotes "macrophages"-, and M denotes "megakaryocytes"-containing colony.

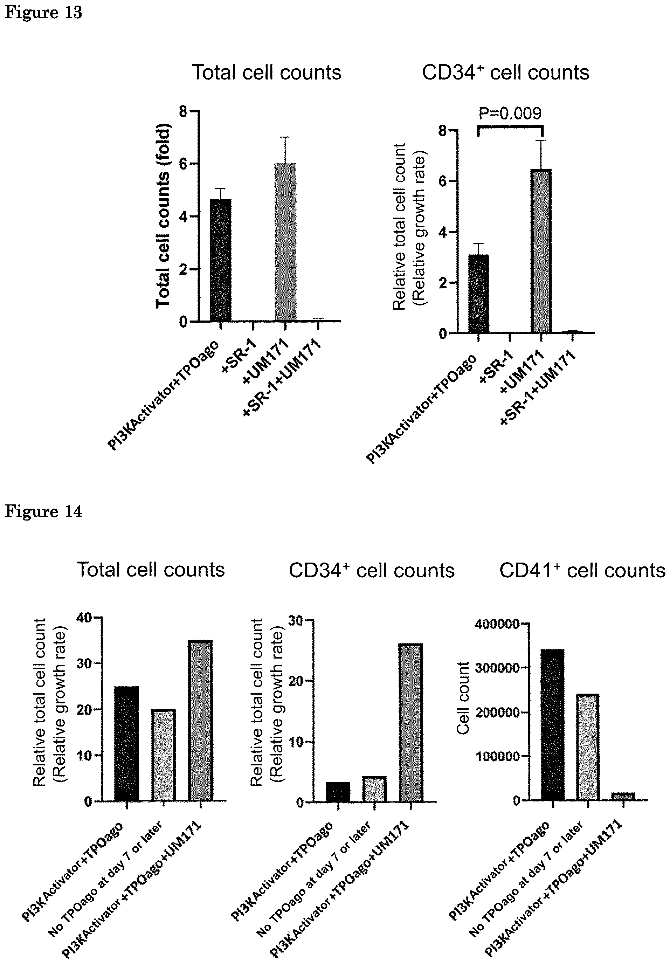

[Figure 13] FIG. 13 shows the growth rate of total cells or CD34⁺ cells at day 14 when human hematopoietic stem cells were cultured in the absence of albumin and in the presence of PVA in a culture medium without SCF or TPO but containing a PI3K activator; a TPO receptor agonist; and SRI and/or UM171.
[Figure 14] FIG. 14 shows the growth rate of total cells or CD34⁺ cells or the number of CD41⁺ cell at day 14 when the respective cells were cultured under conditions 1 to 3.

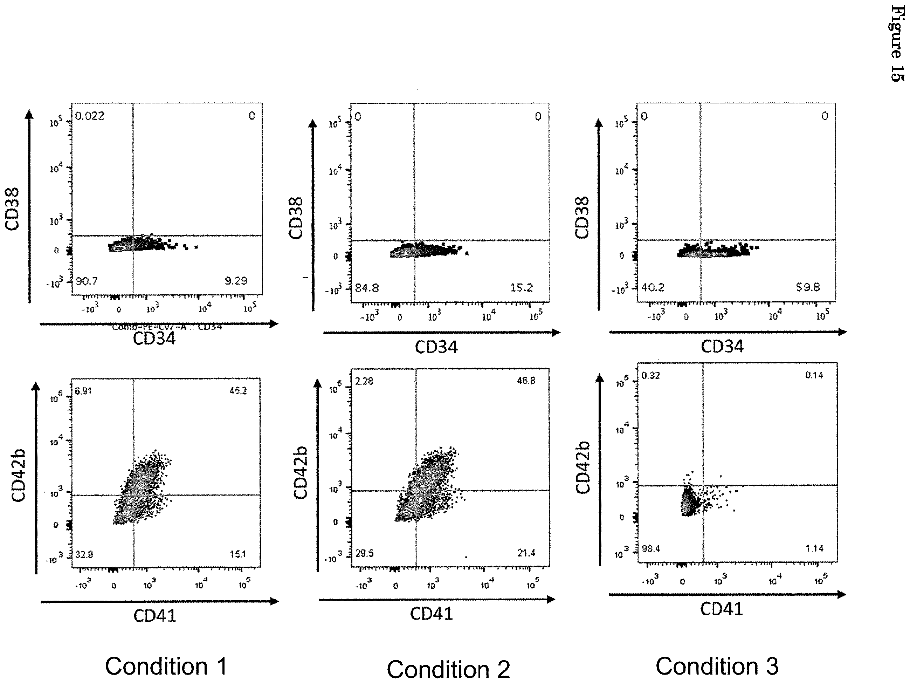

[Figure 15] FIG. 15 shows the results of flow cytometry using cells in the culture at day 14 when the respective cells were culture under conditions 1 to 3. The upper panels show the results using CD34 (abscissa) and CD38 (ordinate), and the lower panels show the results using CD41a (abscissa) and CD42b (ordinate).

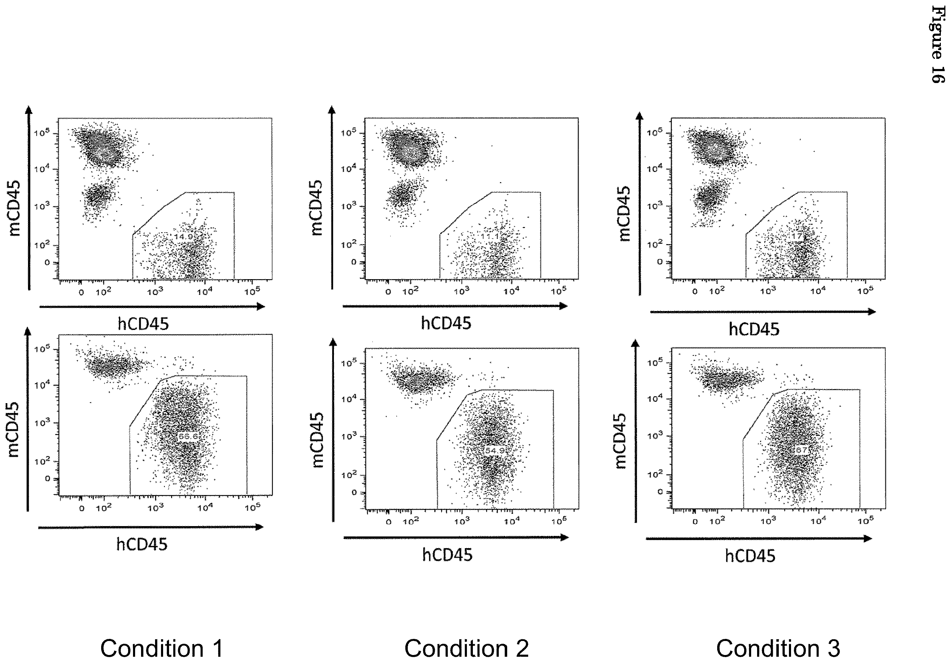

[Figure 16] FIG. 16 is charts indicating engraftment of human hematopoietic stem cells in mouse peripheral blood at 12 weeks after human hematopoietic stem cells were cultured under respective conditions 1 to 3 and then transplanted at day 7 in each mouse.

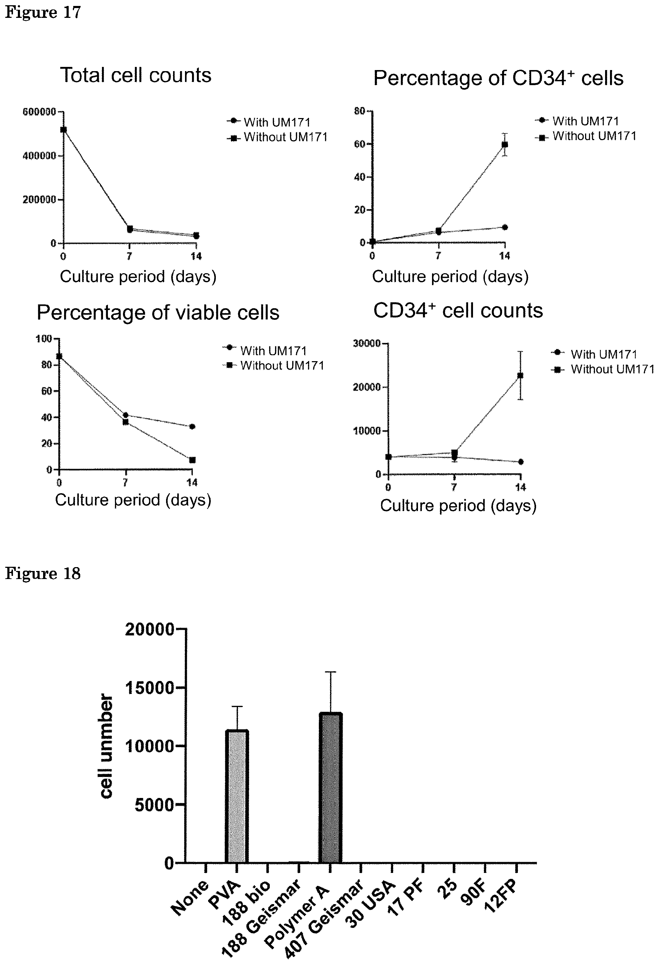

[Figure 17] FIG. 17 shows the number of total cells, the percentage of CD34⁺ cells, the percentage of viable cells, or the number of CD34⁺ cells in a culture obtained after monocytes in given cord blood were separated and cultured for 14 days under conditions in the presence or absence of UM171 in a culture medium without any of albumin, SCF, or TPO but containing PVA, a PI3K activator, and a TPO receptor agonist.
[Figure 18] FIG. 18 shows the results of *in vitro* culture experiments of mouse hematopoietic stem cells in the absence of albumin. In each mouse hematopoietic stem cell culture medium, the indicated component was included at a final concentration of 0.1%. More specifically, used was each culture medium containing, at a final concentration of 0.1%, BASF's Kolliphor (trademark) P188 bio (188 bio), Kolliphor (trademark) P 188 Geismar (188 Geismar), Soluplus (Sol+), Kolliphor (trademark) P 407 Geismar (407 Geismar), Kollidon (trademark) 30 Origin USA (30 USA), Kollidon (trademark) 17 PF (17 PF), Kollidon (trademark) 25 (25), Kollidon (trademark) 90F (90F), or Kollidon (trademark) 12PF (12PF)

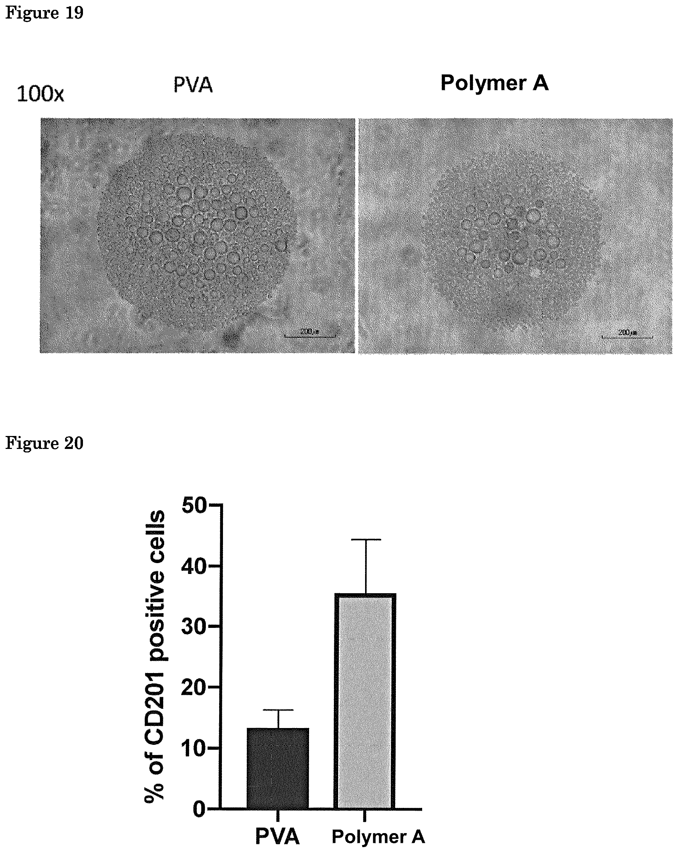

[Figure 19] FIG. 19 shows the results of *in vitro* colony formation assay using mouse hematopoietic stem cells in an albumin-free, serum-free medium in the presence of PVA or polymer A.
[Figure 20] FIG. 20 shows the percentage (%) of a stem cell marker CD201-positive mouse hematopoietic stem cells cultured in an albumin-free, serum-free medium in the presence of PVA or polymer A.

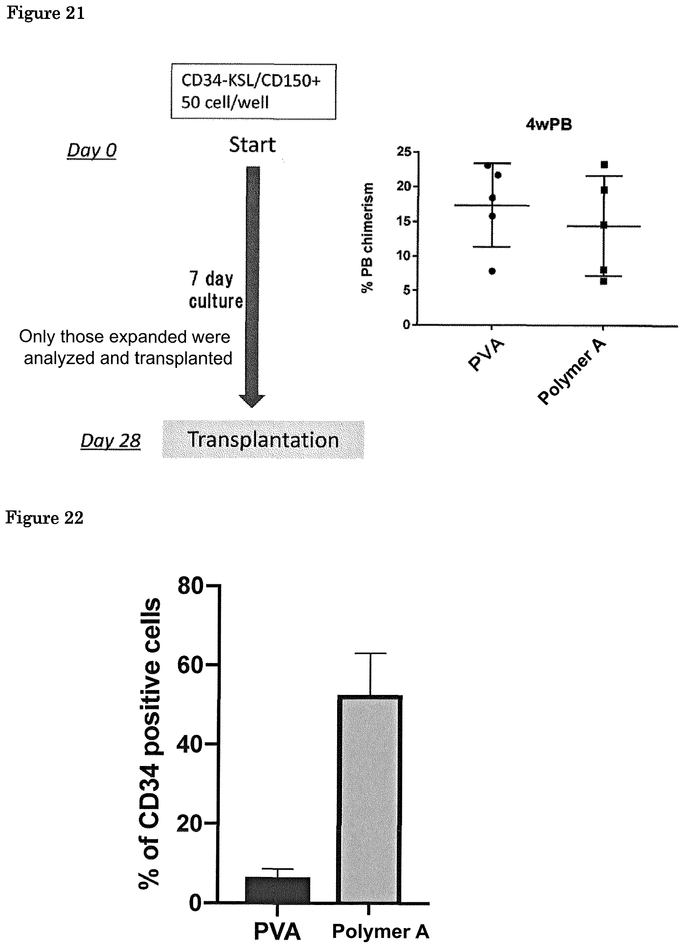

[Figure 21] FIG. 21 shows a scheme (left) in which mouse hematopoietic stem cells cultured in an albumin-free, serum-free medium in the presence of PVA or polymer A were subjected to hematopoietic stem cell transplantation into an irradiated mouse, and the percentage of mouse peripheral blood chimerism 4 weeks after the transplantation. The percentage of chimerism indicates the proportion of cells derived from transplanted hematopoietic stem cells in all the blood cells.
[Figure 22] FIG. 22 indicates the percentage of CD34-positive cells in mouse hematopoietic stem cells cultured in an albumin-free, serum-free medium in the presence of PVA or polymer A.

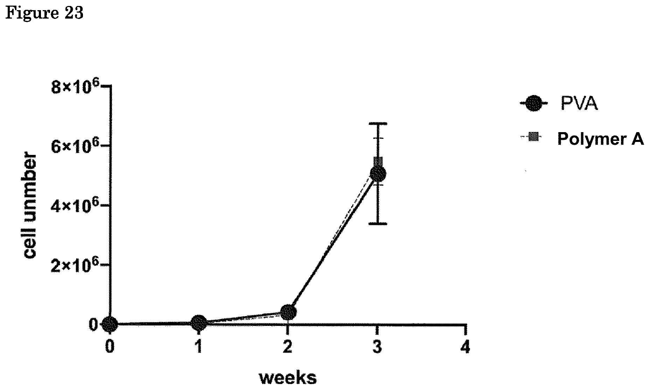

[Figure 23] FIG. 23 indicates a change in the number of mouse hematopoietic stem cells cultured in an albumin-free, serum-free medium in the presence of PVA or polymer A.

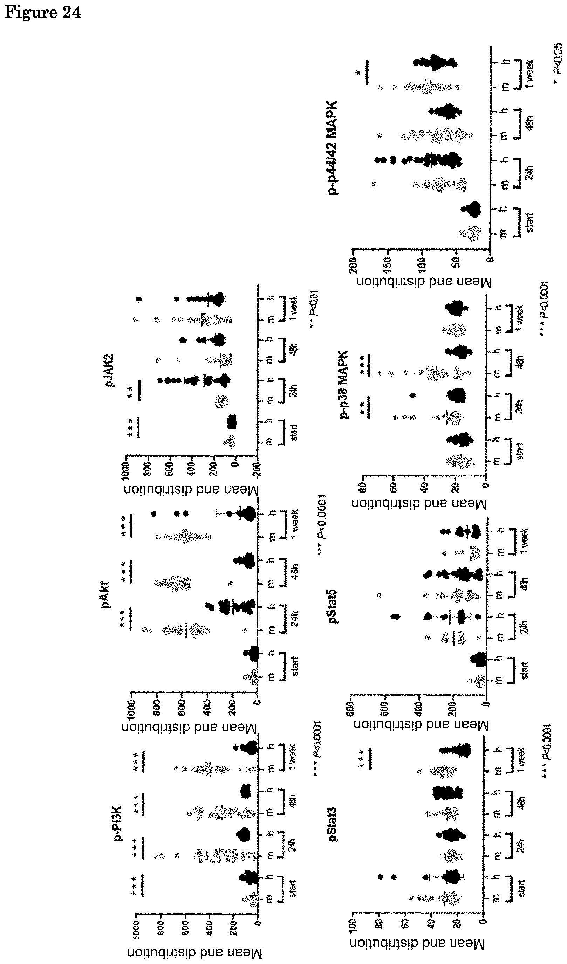

[Figure 24] FIG. 24 is graphs indicating the degree of phosphorylation of each signaling factor downstream of SCF and TPO in mouse or human hematopoietic stem cells in the presence of 10 ng/mL stem cell factor (SCF) and 100 ng/mL thrombopoietin (TPO), in the absence of albumin, and in the presence of PVA. The sign "m" represents mouse hematopoietic stem cells, and the "h" represents human hematopoietic stem cells.

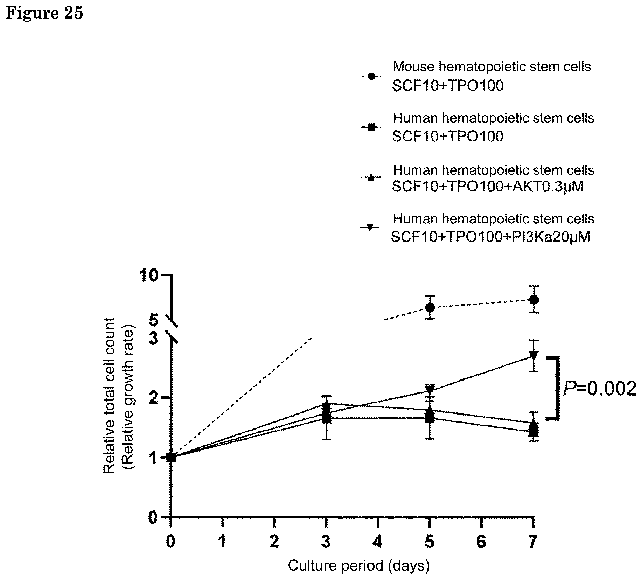

[Figure 25] FIG. 25 shows the results of culturing, in the absence of albumin and in the presence of PVA, human hematopoietic stem cells in a medium containing 10 ng/mL human stem cell factor (SCF) and 100 ng/mL human thrombopoietin in the presence of AKT Activator II (AKTa) or PI3K activator (PI3Ka).

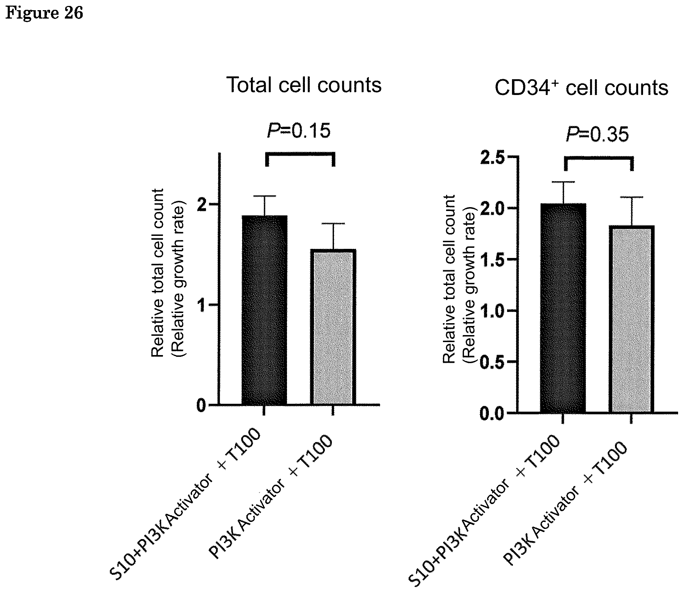

[Figure 26] FIG. 26 shows the growth rate of total cells or CD34+ cells at day 7 after human hematopoietic stem cells were cultured in the absence of albumin and in the presence of PVA and 100 ng/mL human thrombopoietin (TPO). In FIG. 9, SCF-containing conditions and SCF-free conditions are compared. FIG. 4 indicates that no statistically significant difference in the number of total cells or CD34⁺ cells at culture day 7 was found between the two conditions.

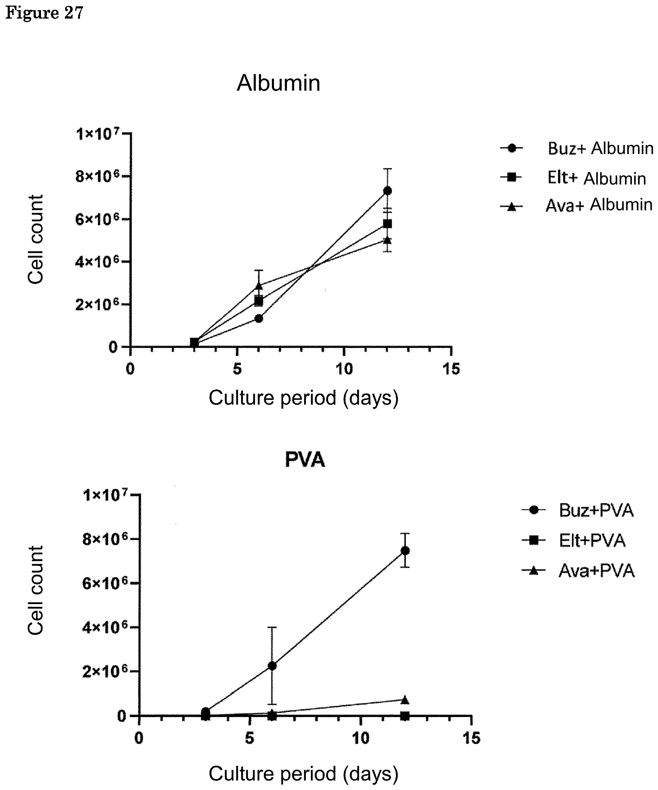

[Figure 27] FIG. 27 shows the time course of the number of cultured human hematopoietic stem cells in the presence of albumin or PVA while TPO was replaced by each TPO receptor agonist. The sign "Buty" represents butyzamide, the "Elt" represents eltrombopag, and the "Ava" represents avatrombopag.

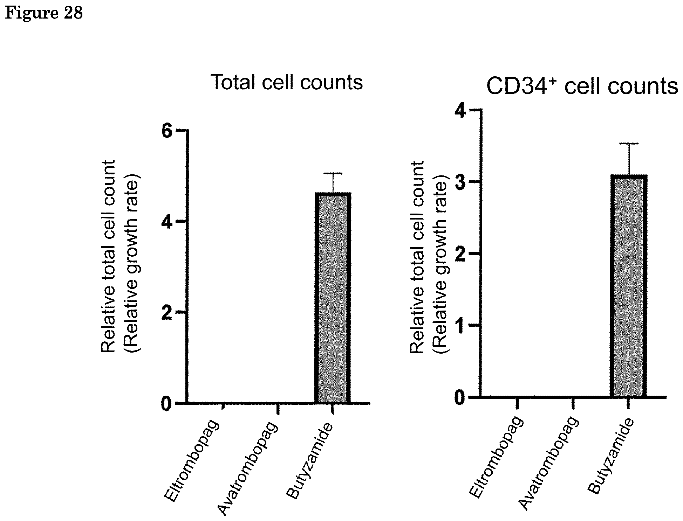

[Figure 28] FIG. 28 shows the growth rate of total cells or CD34⁺ cells at day 7 when human hematopoietic stem cells were cultured in the presence of albumin or PVA while TPO was replaced by each TPO receptor agonist.

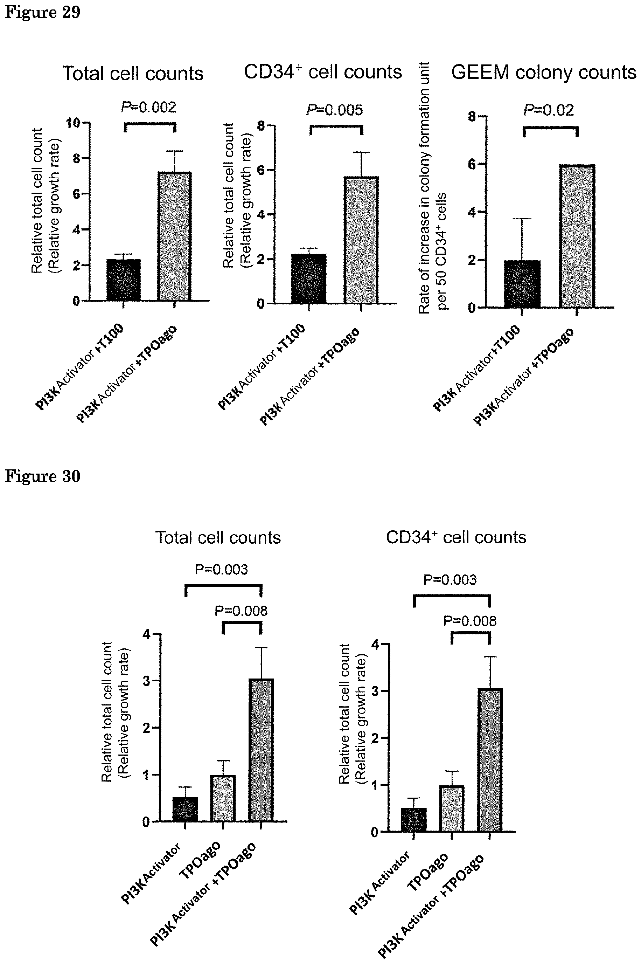

[Figure 29] FIG. 29 indicates the number of total cells, the number of CD34⁺ cells, or the number of GEmM colonies at day 7 when human hematopoietic stem cells were cultured in a culture medium containing a PI3K activator and TPO or butyzamide (Buty) in the absence of albumin and in the presence of PVA. The types of colonies were identified under a microscope after each colony was picked under a microscope, a cytospin sample was prepared, and the sample was subjected to Giemsa staining. G denotes "granulocytes", E denotes "erythroblasts", m denotes "macrophages", and M denotes "megakaryocytes".
[Figure 30] FIG. 30 shows the growth rate of total cells or CD34⁺ cells at day 7 when human hematopoietic stem cells were cultured in the absence of albumin and in the presence of PVA in a culture medium without SCF or TPO but containing a PI3K activator, a TPO receptor agonist, or a combination of them.

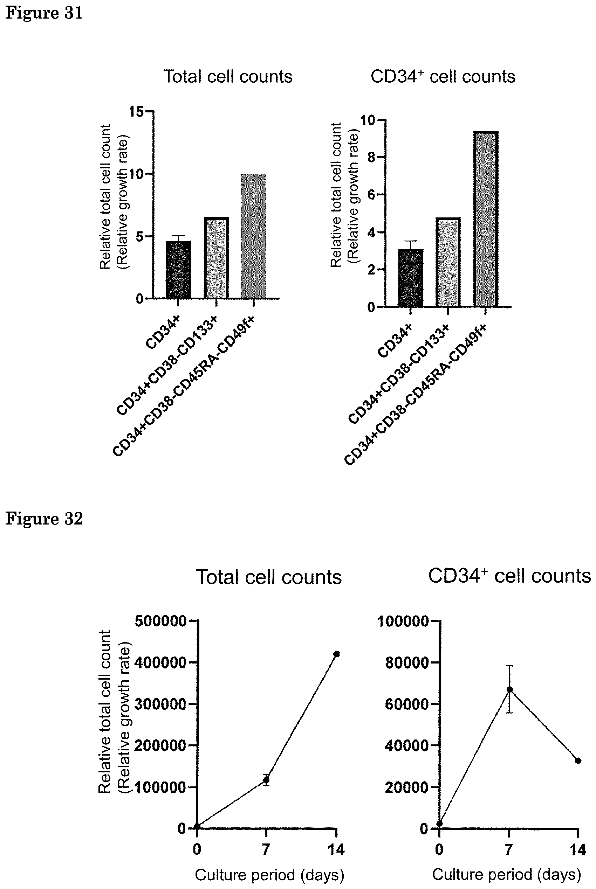

[Figure 31] FIG. 31 shows the growth rate of each cell population in a culture obtained at day 7 when human hematopoietic stem cells were cultured in the absence of albumin and in the presence of PVA in a culture medium without SCF or TPO but containing a PI3K activator and a TPO receptor agonist.
[Figure 32] FIG. 32 shows the time course of the number of total cells or the number of CD34⁺ cells at days 7 and 14 when human hematopoietic stem cells were cultured in the absence of albumin and in the presence of PVA in a culture medium without SCF or TPO but containing a PI3K activator and a TPO receptor agonist.

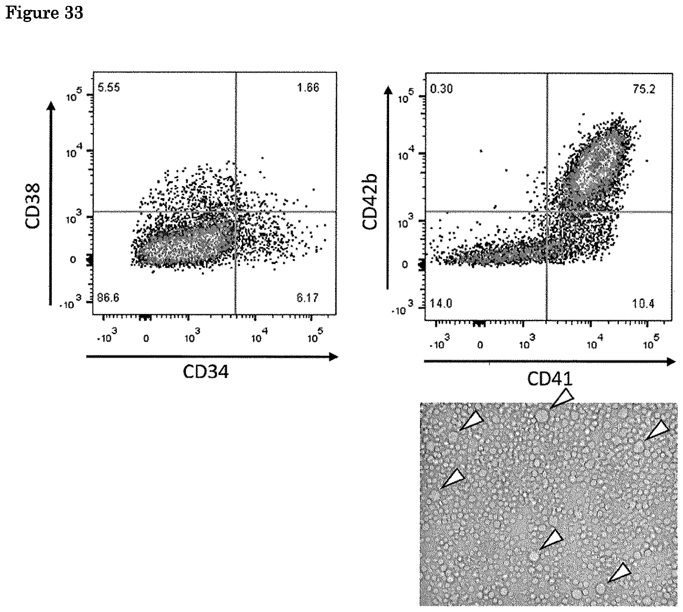

[Figure 33] FIG. 33 shows the results of gated cells obtained at day 14 when human hematopoietic stem cells were cultured in the absence of albumin and in the presence of PVA in a culture medium without SCF or TPO but containing a PI3K activator and a TPO receptor agonist. The left panel shows the results of flow cytometry using CD34 and CD38 as markers, and the right panel shows the results of flow cytometry using CD41a and CD42b as markers. A photograph of FIG. 16 is an optical micrograph of the obtained culture.

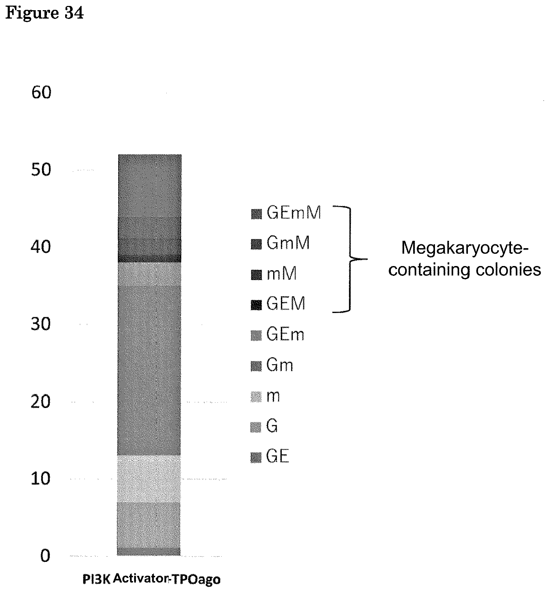

[Figure 34] FIG. 34 shows the results of colony assay of cells obtained at day 14 when human hematopoietic stem cells were cultured in the absence of albumin and in the presence of PVA in a culture medium without SCF or TPO but containing a PI3K activator and a TPO receptor agonist. The types of colonies were identified under a microscope after each colony was picked under a microscope, a cytospin sample was prepared, and the sample was subjected to Giemsa staining. G denotes "granulocytes"-, E denotes "erythroblasts"-, m denotes "macrophages"-, and M denotes "megakaryocytes"-containing colony.

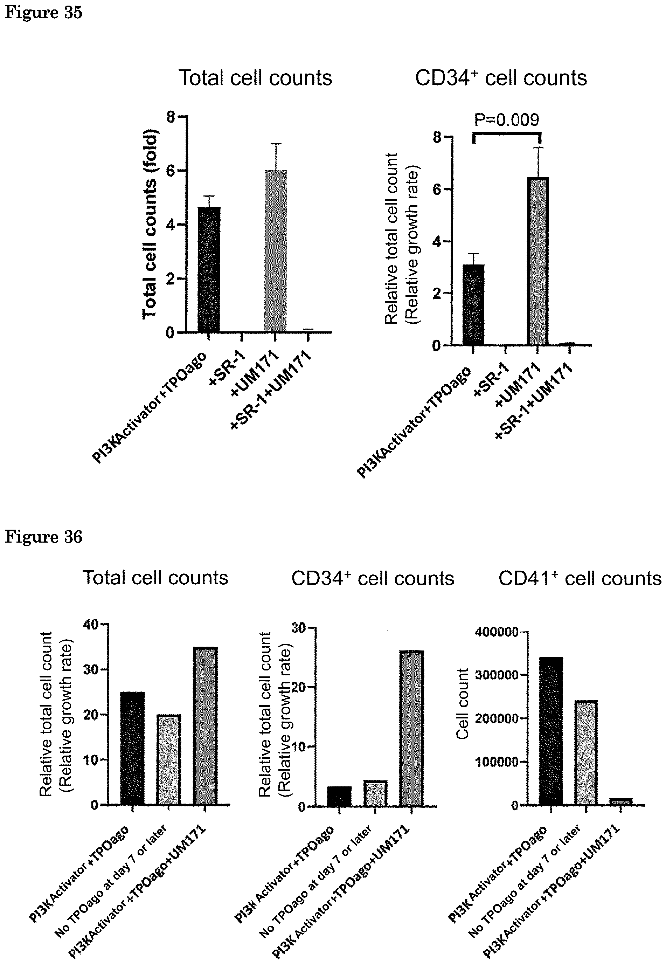

[Figure 35] FIG. 35 shows the growth rate of total cells or CD34⁺ cells at day 14 when human hematopoietic stem cells were cultured in the absence of albumin and in the presence of PVA in a culture medium without SCF or TPO but containing a PI3K activator; a TPO receptor agonist; and SRI and/or UM171.
[Figure 36] FIG. 36 shows the growth rate of total cells or CD34⁺ cells or the number of CD41⁺ cell at day 14 when the respective cells were cultured under conditions 1 to 3.

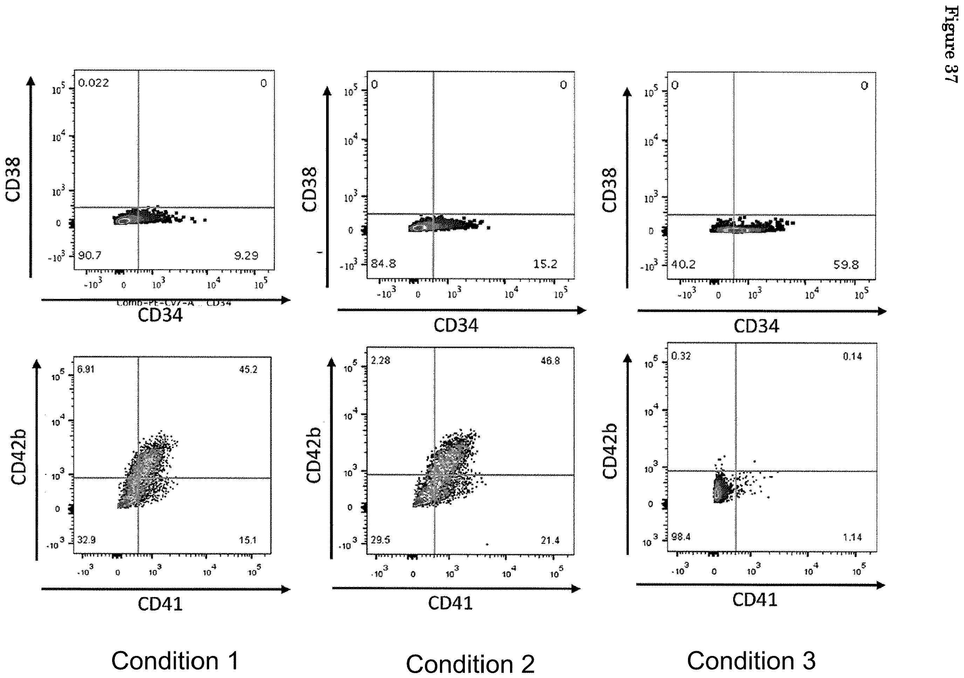

[Figure 37] FIG. 37 shows the results of flow cytometry using cells in the culture at day 14 when the respective cells were culture under conditions 1 to 3. The upper panels show the results using CD34 (abscissa) and CD38 (ordinate), and the lower panels show the results using CD41a (abscissa) and CD42b (ordinate).

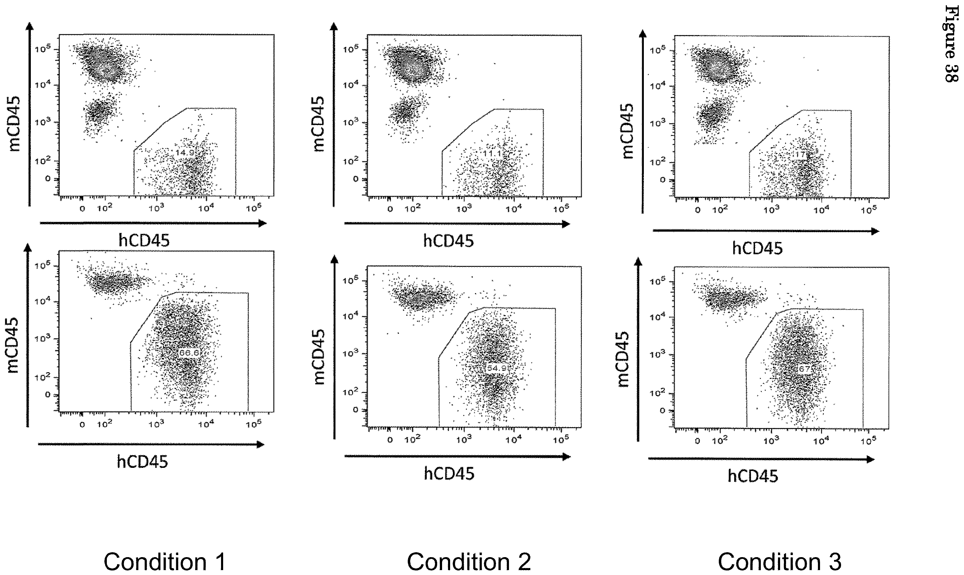

[Figure 38] FIG. 38 is charts indicating engraftment of human hematopoietic stem cells in mouse peripheral blood at 12 weeks after human hematopoietic stem cells were cultured under respective conditions 1 to 3 and then transplanted at day 7 in each mouse.

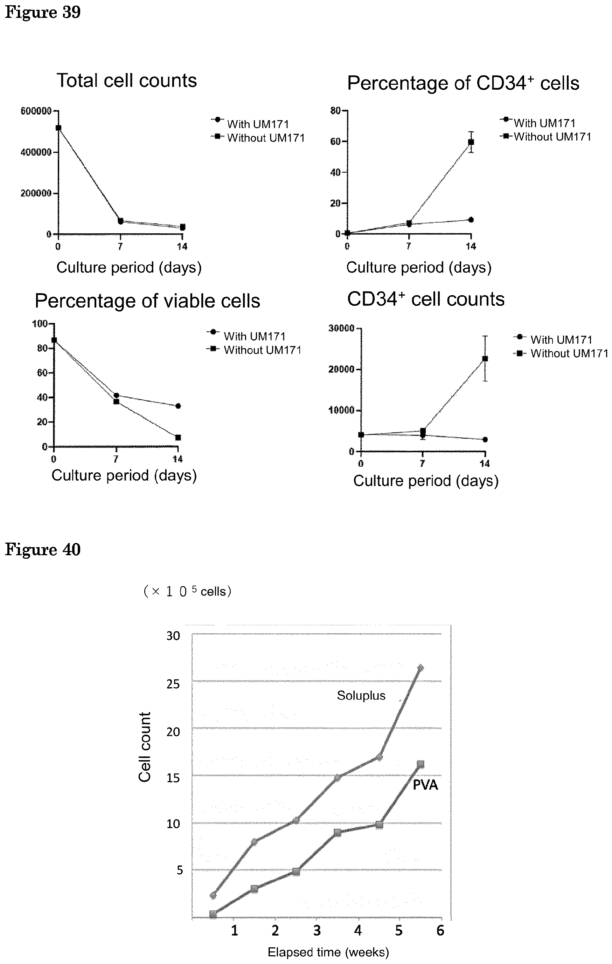

[Figure 39] FIG. 39 shows the number of total cells, the percentage of CD34⁺ cells, the percentage of viable cells, or the number of CD34⁺ cells in a culture obtained after monocytes in given cord blood were separated and cultured for 14 days under conditions in the presence or absence of UM171 in a culture medium without any of albumin, SCF, or TPO but containing PVA, a PI3K activator, and a TPO receptor agonist.
[Figure 40] FIG. 40 shows the growth of human T cells in an albumin-free, cytokine-free medium in the presence of PVA or Soluplus (trademark).

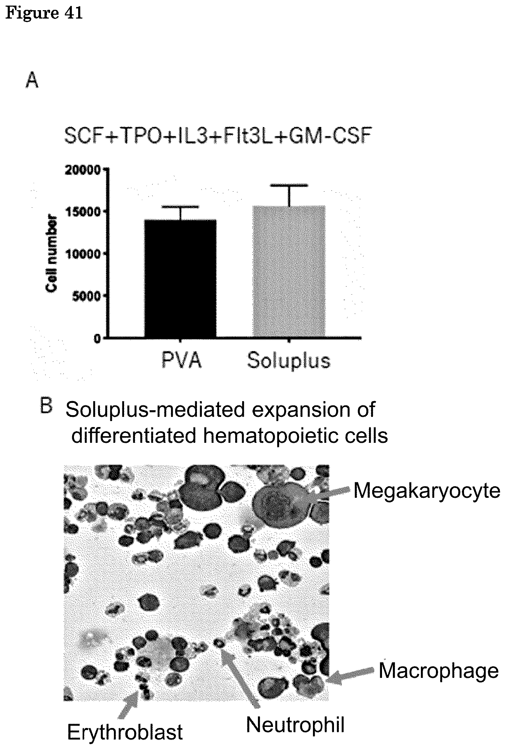

[Figure 41] FIG. 41 shows the results of human hematopoietic stem cell differentiation experiments in an albumin-free medium in the presence of PVA or Soluplus (trademark). The hematopoietic cells are demonstrated to proliferate and differentiate (in particular, proliferate) into a wide range of blood cell lineages.

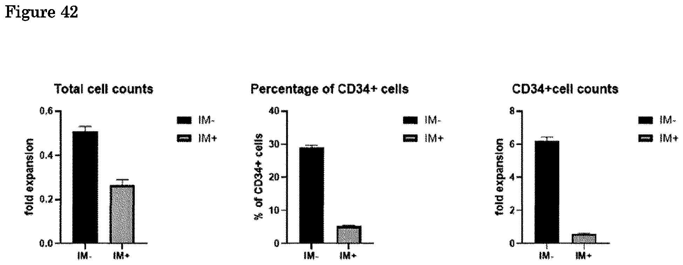

[Figure 42] FIG. 42 shows the growth potential of chronic myeloid leukemia (CML) cells in an albumin-free, cytokine-free medium in the presence of PVA or Soluplus (trademark). In the graphs, "IM+" means the presence of imatinib, and "IM-" means the absence of imatinib.

### Description of Embodiments

As used herein, the "hematopoietic stem cells" refer to stem cells capable of differentiating into blood cells. Hematopoietic stem cells can be collected from bone marrow, umbilical cord, placenta, or peripheral blood. Human hematopoietic stem cells are CD34-positive cells. In humans, hematopoietic stem cells are known to be abundant in the CD34-positive, CD38-negative cell fraction. Thus, human hematopoietic stem cells can be CD34-positive and CD38-negative. Hematopoietic stem cells may be cells obtained by *ex vivo* differentiation of pluripotent stem cells such as ES cells or iPS cells.

As used herein, "blood cells" are hematopoietic stem cells or cells differentiated from hematopoietic stem cells. Blood cells are largely classified into hematopoietic stem cells, hematopoietic progenitors, and hematopoietic cells according to their differentiation stage. Hematopoietic stem cells differentiate via hematopoietic progenitors into hematopoietic cells. More specifically, hematopoietic stem cells can differentiate via lymphoblasts into lymphocytes (e.g., T cells, B cells, NK cells). Hematopoietic stem cells can also differentiate via hematopoietic progenitors and monoblasts into monocytes. Hematopoietic stem cells can also differentiate via hematopoietic progenitors, myeloblasts, promyelocytes, myelocytes, postmyelocytes, and rod-shaped nucleated cells into neutrophils. Hematopoietic stem cells can also differentiate via hematopoietic progenitors and myeloblasts into granulocytic leukocytes, such as eosinophils or basophils, or macrophages. Hematopoietic stem cells can also differentiate via hematopoietic progenitors, proerythroblasts, basophilic erythroblasts, polychromatic erythroblasts, and orthochromatic erythroblasts into erythrocytes. Hematopoietic stem cells can also produce platelets after undergoing through hematopoietic progenitors, pro-megakaryocytes, and megakaryocytes. All the cells that can be differentiated from hematopoietic stem cells are blood cells. Examples of the blood cells include cancer cells or non-cancer cells. Examples of the cancer cells include chronic myelogenous leukemia (CML) cells or CML stem cells.

As used herein, the term "*ex vivo*" means outside the body. As used herein, the term "*ex vivo*" is used in contrast to *in vivo* (inside the body), and means that cells present inside the body are taken outside the body from the inside. The culturing may be performed *ex vivo.*

As used herein, the "positive" means that the cell is found to express a molecule identified by the immediately preceding term. As used herein, "positive" is sometimes simply denoted as "⁺".

As used herein, "polyvinyl alcohol" (PVA) means a polymer of vinyl alcohol. Polyvinyl alcohol can be obtained by saponification of polyvinyl acetate, which is obtained by polymerizing a vinyl acetate monomer. The weight-average molecular weight (MW) of polyvinyl alcohol may be, for example, 1 kDa to 20 kDa, 3 kDa to 17 kDa, 5 kDa to 15 kDa, or 7 kDa to 13 kDa. In the case of saponification of polyvinyl acetate to obtain PVA by the above method, the rate of saponification may be 80% or more, 85% or more, 90% or more, 95% or more, or 99% or more.

As used herein, the "albumin" is a protein known as a plasma component. Albumin is said to account for 60% of plasma proteins and is abundantly present in blood, serum, and plasma. Albumin is thought to be responsible for maintaining the osmotic pressure of blood *in vivo* and for transporting biological substances such as fatty acids and hormones by binding to them. Serum albumin is also known to be important in the maintenance and culture of hematopoietic stem cells. Human serum albumin (hereinafter sometimes referred to as "HSA") is human serum albumin, which can be, for example, a protein having the amino acid sequence registered as GenBank Accession No: AAN17825.1 or human serum albumin having the corresponding amino acid sequence.

As used herein, the "agonist" refers to a substance that can activate a protein such as a receptor or enzyme.

As used herein, the "PI3K" means phosphatidylinositol 3-kinase. PI3K is an enzyme that can phosphorylate inositol phospholipids, a component of cells. Phosphorylated phosphatidylinositol 3,4,5-trisphosphate (PIP3) mediates Akt (also known as protein kinase B) phosphorylation and transduces downstream signaling. As used herein, the "PI3K activator" means an agonist of receptor tyrosine kinase and a substance that can activate PI3K. The PI3K activator can be selective for PI3K.

As used herein, the "TPO" means thrombopoietin. TPO is a protein responsible for the differentiation of hematopoietic stem cells into megakaryocytes. TPO is known to be formally involved in the maintenance of hematopoietic stem cells. Human thrombopoietin is, for example, a protein having the amino acid sequence registered as GenBank Accession No: AAB33390.1 or thrombopoietin having the corresponding amino acid sequence.

As used herein, the "TPO receptor agonist" means a substance, other than TPO, that can activate a TPO receptor. Examples of the TPO receptor agonist include each TPO variant other than TPO, each peptide, or each compound that can activate a TPO receptor. As used herein, the "compound" is a concept that encompasses organic compounds. The TPO receptor agonist can be selective for a TPO receptor.

As used herein, stem cell factor (SCF) is a hematopoietic cell growth factor that acts in the early stages of hematopoiesis. Human stem cell factor is, for example, a protein having the amino acid sequence registered as GenBank Accession No: AAA85450.1 or SCF having the corresponding amino acid sequence.

As used herein, the "culturing" means incubating cells under conditions suitable for their growth or maintenance. The incubation can be performed, in the case of human cells, under an atmosphere preferably at 37°C and 5% CO₂. If the "culturing" involves proliferation, the "culturing" is understood to involve the production of expanded cells. As used herein, the "culturing" can be performed in a serum-free medium. As used herein, the "culturing" can be performed in a chemically defined culture medium (or in a completely synthetic medium). The chemically defined culture medium is a serum-free medium.

As used herein, the "culture medium" means a medium used for culturing cells. The culture medium may be prepared by adding components essential to a basic medium. The essential components may include a pH modifier, a sugar source such as glucose, antibiotics (e.g., penicillin and streptomycin), and essential amino acids such as glutamine, as well as culture additives such as insulin, transferrin, selenium (e.g., sodium selenite), and/or ethanolamine.

As used herein, the term "free" means that some material is not contained at concentrations above the detection limit or not contained at all. For example, a serum-free or albumin-free, serum-free medium is albumin-free and free of serum or albumin. The term "albumin-free" means that albumin is not contained at concentrations at or above the detection limit or not contained at all. The term "cytokine-free" means that any cytokine is not contained at concentrations at or above the detection limit or not contained at all. The medium may be albumin-free. The medium may be cytokine-free. The medium may be albumin-free and cytokine-free.

As used herein, the "cytotoxicity" means the characteristic of exerting the effect of decreasing the number of cells or killing cells during culture. As used herein, "non-cytotoxic" refers to the characteristic of not decreasing the number of cells during culture. An increased concentration of some substance may cause cytotoxicity. In this case, even if the concentration is reduced to a level that does not cause cytotoxicity, the expected effect of the substance may still occur. This case can be defined as non-cytotoxic. As used herein, a medium free of any cytotoxic agent or agent having cytotoxicity means that the medium does not contain any cytotoxic agent or agent having cytotoxicity in sufficient amounts to cause cytotoxicity. Accordingly, when an agent is cytotoxic at high concentrations and is used at concentrations that cause no cytotoxicity, the agent is neither a cytotoxic agent nor an agent having cytotoxicity.

The present inventors have found and reported that mouse hematopoietic stem cells (sometimes referred to as KSL cells due to their isolation method) can be grown in large numbers over a long period of time by adding polyvinyl alcohol (PVA) to an albumin-free serum-free medium (Wilkinson et al., Nature, 571: 117-121, 2019). However, no method has been established for expansion of human hematopoietic stem cells in an albumin-free, serum-free medium. Now, the present inventors have discovered that human hematopoietic stem cells can markedly proliferate while their stem cell properties are maintained in the presence of polyethylene glycol modified with a copolymer containing a polyvinylcaprolactam block and a polyvinylacetate block. The present inventors have also found that in human hematopoietic stem cells cultured in the presence of PVA, SCF, and TPO, even in an albumin-free, serum-free medium, the activation of various signaling pathways, including the PI3K and Akt pathways, is weak. The present inventors have also found that human hematopoietic stem cells and human blood cells can proliferate by the addition of a PI3K activator in the presence of PVA, SCF and TPO, even in an albumin-free, serum-free medium. The present inventors have further found that a PI3K activator can completely replace SCF in the presence of PVA, even in an albumin-free, serum-free medium. Furthermore, the present inventors have found that TPO can be replaced by a TPO receptor agonist in the presence of PVA and a PI3K activator, even in an albumin-free, serum-free medium. Still furthermore, the present inventors have found that human hematopoietic stem cells or human blood cells can favorably proliferate in the presence of polyethylene glycol modified with a copolymer containing a polyvinylcaprolactam block and a polyvinylacetate block, a PI3K activator, and a TPO receptor agonist, even in an albumin-free, serum-free medium. At that time, it is unnecessary to add SCF and TPO to the medium. Moreover, the present inventors have found that human hematopoietic stem cells can be maintained and expanded for a long period of time by adding UM171 to the medium.

The present invention provides a method of culturing human cells, comprising:
culturing human cells in a culture medium, wherein
the culture medium is a serum albumin-free medium and comprises an additive, the additive being selected from the group consisting of polyvinyl alcohol and modified polyalkylene glycol, and further comprises
   (1) a phosphatidylinositol 3-kinase (PI3K) activator and at least one compound selected from the group consisting of thrombopoietin (TPO) and TPO receptor agonists;
   (2) a TPO receptor agonist and at least one compound selected from the group consisting of stem cell factor (SCF) and PI3K activators; or
   (3) a PI3K activator and a TPO receptor agonist, so that
the culturing allows the number of human hematopoietic stem cells or human blood cells to increase or be maintained.

The human cells may be cells selected from the group consisting of human hematopoietic stem cells and human blood cells. In addition, the human cells may also be blood cells other than hematopoietic stem cells. The blood cells may be cells selected from the group consisting of hematopoietic progenitors and hematopoietic cells. The blood cells may be one or more cells selected from the group consisting of lymphoblasts, lymphocytes (e.g., T cells, B cells, NK cells, and NKT cells), CD3-positive cells, myeloblasts, promyelocytes, myelocytes, postmyelocytes, rod-shaped nucleated cells, and neutrophils, myeloblasts, granulocytes (e.g., eosinophils and basophils), macrophages, proerythroblasts, basophilic erythroblasts, polychromatic erythroblasts, orthochromatic erythroblasts, erythrocytes as well as pro-megakaryocytes and megakaryocytes. The human cells may be, for example, one or more cells selected from the group consisting of chronic myelogenous leukemia (CML) cells and CML stem cells.

In a certain embodiment, the additive may be an additive that can replace albumin. The additive is, in a certain embodiment, modified polyalkylene glycol. In a certain embodiment, the modified polyalkylene glycol may be, for example, modified polyethylene glycol. The modified polyalkylene glycol or modified polyethylene glycol may be preferably modified with a copolymer containing a polyvinylcaprolactam block and a polyvinylacetate block.

In a certain embodiment, the additive may be polyvinyl alcohol.

According to the invention, PVA or modified polyalkylene glycol may be added. This reduces the amount of albumin, such as serum albumin, added to a culture medium, preferably making the culture medium albumin-free. In a certain embodiment, the culture medium can be a serum-free medium, for instance, an albumin-free, serum-free medium.

According to the present invention, a PI3K activator can replace SCF. Meanwhile, in the invention, a TPO receptor agonist can replace TPO. In this way, in the invention, the amount of SCF or TPO added to the culture medium can be reduced and, preferably, the culture medium can be made cytokine-free. In a certain embodiment, the culture medium may be a serum-free medium, for instance, a cytokine-free, serum-free medium.

In a certain embodiment, PVA or modified polyalkylene glycol may be added to a culture medium. This can reduce the amount of albumin, such as serum albumin, added to the culture medium. In addition, a PI3K activator and a TPO receptor agonist may be added to the culture medium. This can reduce the amounts of SCF and TPO added to the culture medium. In a certain embodiment, the culture medium may be an albumin-free, cytokine-free, serum-free medium.

Another embodiment of the invention provides a method of culturing human hematopoietic stem cells or human blood cells, comprising:
culturing the human hematopoietic stem cells or human blood cells in a culture medium, wherein
the culture medium comprises polyvinyl alcohol, does not comprise any albumin, and comprises:
   (1) a phosphatidylinositol 3-kinase (PI3K) activator and at least one compound selected from the group consisting of thrombopoietin (TPO) and TPO receptor agonists;
   (2) a TPO receptor agonist and at least one compound selected from the group consisting of stem cell factor (SCF) and PI3K activators; or
   (3) a PI3K activator and a TPO receptor agonist.

Another embodiment of the invention provides a method of culturing human hematopoietic stem cells or human blood cells in a culture medium, comprising:
bringing, in the culture medium, the human hematopoietic stem cells or human blood cells into contact with polyvinyl alcohol and
bringing, in the culture medium, the human hematopoietic stem cells or human blood cells into contact with:
   (1) a phosphatidylinositol 3-kinase (PI3K) activator and at least one compound selected from the group consisting of thrombopoietin (TPO) and TPO receptor agonists;
   (2) a TPO receptor agonist and at least one compound selected from the group consisting of stem cell factor (SCF) and PI3K activators; or
   (3) a PI3K activator and a TPO receptor agonist.

In this embodiment, the culturing allows the number of human hematopoietic stem cells or human blood cells to increase or be maintained.

In the invention, the PI3K activator used is not cytotoxic to human hematopoietic stem cells or human blood cells in the absence of albumin and in the presence of PVA. In other words, the PI3K activator used in the invention is non-cytotoxic.

In addition, the TPO receptor agonists used in the invention are non-cytotoxic to human hematopoietic stem cells or human blood cells in the absence of albumin and in the presence of PVA. Whether or not the PI3K activators are non-cytotoxic can be checked, if appropriate, using a test of culturing human hematopoietic stem cells or human blood cells, by those skilled in the art. Here, the test of culturing human hematopoietic stem cells or human blood cells can be implemented using IMDM medium containing 1% insulin-transferrin-selenium, 1% penicillin-streptomycin-glutamine, 100 ng/mL TPO, and 0.1% PVA. Whether or not the TPO receptor agonists are non-cytotoxic can be checked, if appropriate, using a test of culturing human hematopoietic stem cells or human blood cells, by those skilled in the art. Here, the test of culturing human hematopoietic stem cells or human blood cells can be implemented using IMDM medium containing 1% insulin-transferrin-selenium, 1% penicillin-streptomycin-glutamine, 10 ng/mL SCF, and 0.1% PVA.

In a certain embodiment of the invention, the culture medium used in the culture method of the invention may be a culture medium for human hematopoietic stem cells or human blood cells, the medium comprising:
(1) a PI3K activator and at least one compound selected from the group consisting of TPO and TPO receptor agonists;
(2) a TPO receptor agonist and at least one compound selected from the group consisting of stem cell factor (SCF) and PI3K activators; or
(3) a PI3K activator and a TPO receptor agonist.

In a certain embodiment of the invention, the culture medium used in the culture method of the invention may be a culture medium for human hematopoietic stem cells or human blood cells, the medium comprising:
polyvinyl alcohol or modified polyalkylene glycol and
(1) a PI3K activator and at least one compound selected from the group consisting of TPO and TPO receptor agonists;
(2) a TPO receptor agonist and at least one compound selected from the group consisting of stem cell factor (SCF) and PI3K activators; or
(3) a PI3K activator and a TPO receptor agonist.

In a certain embodiment of the invention, the culture medium used in the culture method of the invention may be a culture medium for human hematopoietic stem cells or human blood cells, the medium being albumin (especially serum albumin)-free, and the medium comprising:
(1) a PI3K activator and at least one compound selected from the group consisting of TPO and TPO receptor agonists;
(2) a TPO receptor agonist and at least one compound selected from the group consisting of stem cell factor (SCF) and PI3K activators; or
(3) a PI3K activator and a TPO receptor agonist.

In a certain embodiment of the invention, the culture medium used in the culture method of the invention may be a culture medium for human hematopoietic stem cells or human blood cells, the medium comprising:
polyvinyl alcohol, but no albumin, and
(1) a PI3K activator and at least one compound selected from the group consisting of TPO and TPO receptor agonists;
(2) a TPO receptor agonist and at least one compound selected from the group consisting of stem cell factor (SCF) and PI3K activators; or
(3) a PI3K activator and a TPO receptor agonist.

The culture medium in the invention may contain an effective amount of non-cytotoxic PI3K activator and an effective amount of PVA to expand human hematopoietic stem cells or human blood cells in the absence of albumin. The culture medium in the invention may further contain SCF and/or TPO. The culture medium in the invention may contain an effective amount of non-cytotoxic TPO receptor agonist instead of TPO. The culture medium in the invention may contain an effective amount of non-cytotoxic PI3K activator, an effective amount of PVA, an effective amount of TPO and/or non-cytotoxic TPO receptor agonist, and UM171.

According to the invention, the method of culturing human hematopoietic stem cells may be a method of producing megakaryocyte lineage cells from human hematopoietic stem cells. In this embodiment, the culture medium used in the culture method may be a culture medium containing a PI3K activator and a TPO receptor agonist, wherein the PI3K activator is 740Y-P and the TPO receptor agonist is butyzamide. In this particular embodiment, the culture medium may be free of UM171.

In a certain embodiment of the invention, the culture method of the invention may comprise culturing in the presence of 4-N-[2-benzyl-7-(2-methyltetrazol-5-yl)-9H-pyrimid[4,5-b]indol-4-yl]cyclohexane-1,4-diamine (UM171). In a certain embodiment of the invention, the culture medium used in the culture method of the invention may further comprise UM171. This may facilitate the maintenance of human hematopoietic stem cell characteristics or inhibit differentiation into cells of the megakaryocyte lineage (e.g., megakaryocyte progenitors and megakaryocytes).

In a certain embodiment of the invention, the culture method of the invention does not comprise culturing in the presence of 4-N-[2-benzyl-7-(2-methyltetrazol-5-yl)-9H-pyrimid[4,5-b]indol-4-yl]cyclohexane-1,4-diamine (UM171). In a certain embodiment of the invention, the culture medium used in the culture method of the invention does not further comprise UM171. This facilitates the differentiation of human hematopoietic stem cells into cells of the megakaryocyte lineage (e.g., megakaryocyte progenitors and megakaryocytes).

Another embodiment of the invention provides a method of culturing human hematopoietic stem cells or human blood cells, comprising:
culturing the human hematopoietic stem cells or human blood cells in a culture medium, wherein
the culture medium comprises polyethylene glycol modified with a copolymer containing a polyvinylcaprolactam block and a polyvinylacetate block.

In this method, the medium for the human hematopoietic stem cells or human blood cells may be albumin-free. In this method, the culture medium may further comprise:
(1) a phosphatidylinositol 3-kinase (PI3K) activator and at least one compound selected from the group consisting of thrombopoietin (TPO) and TPO receptor agonists;
(2) a TPO receptor agonist and at least one compound selected from the group consisting of stem cell factor (SCF) and PI3K activators; or
(3) a PI3K activator and a TPO receptor agonist. In this embodiment, the culturing allows the number of human hematopoietic stem cells or human blood cells to increase or be maintained.

Another embodiment of the invention provides a method of culturing human hematopoietic stem cells or human blood cells in a culture medium, comprising:
bringing, in the culture medium, the human hematopoietic stem cells or human blood cells into contact with polyethylene glycol modified with a copolymer containing a polyvinylcaprolactam block and a polyvinylacetate block. In this method, the medium for the human hematopoietic stem cells or human blood cells may be albumin-free. This method further comprises:
bringing, in the culture medium, the human hematopoietic stem cells or human blood cells into contact with:
(1) a phosphatidylinositol 3-kinase (PI3K) activator and at least one compound selected from the group consisting of thrombopoietin (TPO) and TPO receptor agonists;
(2) a TPO receptor agonist and at least one compound selected from the group consisting of stem cell factor (SCF) and PI3K activators; or
(3) a PI3K activator and a TPO receptor agonist.

In this embodiment, the culturing allows the number of human hematopoietic stem cells or human blood cells to increase or be maintained.

The present inventors have revealed that some compounds, including several TPO receptor agonists, can be cytotoxic to human hematopoietic stem cells or human blood cells in the absence of albumin and in the presence of PVA.

In the invention, the PI3K activator herein is non-cytotoxic to human hematopoietic stem cells or human blood cells in the absence of albumin and in the presence of PVA.

In addition, the TPO receptor agonist used herein is non-cytotoxic to human hematopoietic stem cells or human blood cells in the absence of albumin and in the presence of PVA. Whether or not the PI3K activator is non-cytotoxic can be checked, if appropriate, using a test of culturing human hematopoietic stem cells or human blood cells, by those skilled in the art. Here, the test of culturing human hematopoietic stem cells or human blood cells can be implemented using IMDM medium containing 1% insulin-transferrin-selenium, 1% penicillin-streptomycin-glutamine, 100 ng/mL TPO, and 0.1% PVA. Whether or not the TPO receptor agonist is non-cytotoxic can be checked, if appropriate, using a test of culturing human hematopoietic stem cells or human blood cells, by those skilled in the art. Here, the test of culturing human hematopoietic stem cells or human blood cells can be implemented using IMDM medium containing 1% insulin-transferrin-selenium, 1% penicillin-streptomycin-glutamine, 10 ng/mL SCF, and 0.1% PVA.

In a certain embodiment of the invention, the PI3K activator may be a peptide represented by the amino acid sequence RQIKIWFQNRRMKWKKSDGGYMDMS, where Y can be phosphorylated (also called "740Y-P").

In a certain embodiment of the invention, the TPO receptor agonist may be 3-[4-[[[4-[2-methoxy-3-(1-tert-butyl-2-oxapentan-1-yl)phenyl]thiazol-2-yl]amino]carbonyl] -2,6-dichlorophenyl] -2-methylpropenoic acid (hereinafter, sometimes referred to as "butyzamide").

In a certain embodiment of the invention, the PI3K activator is 740Y-P, and the TPO receptor agonist is butyzamide.

In a certain embodiment of the invention, the culture medium used in the culture method of the invention may be a serum-free medium. In a certain embodiment of the invention, the culture medium used in the culture method of the invention may be a chemically defined medium. In a certain embodiment of the invention, the culture medium used in the culture method of the invention is an albumin-free, cytokine-free, or albumin-free and cytokine-free medium (preferably a serum-free medium and more preferably a chemically defined medium). In a certain embodiment of the invention, the culture medium used in the culture method of the invention may be a serum-free medium (preferably a chemically defined medium) that is an albumin-free medium. In a certain embodiment of the invention, the culture medium used in the culture method of the invention may be a serum-free medium (preferably a chemically defined medium) that is a cytokine-free medium. In a certain embodiment of the invention, the culture medium used in the culture method of the invention may be a serum-free medium (preferably a chemically defined medium) that is an albumin-free, cytokine-free medium. In a certain embodiment of the invention, the culture medium used in the culture method of the invention may contain a PI3K activator and a TPO receptor agonist. In a certain embodiment of the invention, the culture medium used in the culture method of the invention is a serum-free medium (preferably a chemically defined medium) that is an albumin-free and cytokine-free medium and that contains polyvinyl alcohol, a PI3K activator, and a TPO receptor agonist. In a certain embodiment of the invention, the culture medium used in the culture method of the invention is a serum-free medium (preferably a chemically defined medium) that contains polyethylene glycol modified with a copolymer containing a polyvinylcaprolactam block and a polyvinylacetate block. In a certain embodiment of the invention, the culture medium used in the culture method of the invention is a serum-free medium (preferably a chemically defined medium) that may contain: polyethylene glycol modified with a copolymer containing a polyvinylcaprolactam block and a polyvinylacetate block; a PI3K activator instead of SCF; and a TPO receptor agonist instead of TPO. In a certain embodiment of the invention, the culture medium used in the culture method of the invention is an albumin-free, cytokine-free, serum-free medium (preferably a chemically defined medium) that may contain: polyethylene glycol modified with a copolymer containing a polyvinylcaprolactam block and a polyvinylacetate block; a PI3K activator; and a TPO receptor agonist. In a certain embodiment of the invention, the culture medium used in the culture method of the invention is a serum-free medium (preferably a chemically defined medium) that may contain: polyethylene glycol modified with a copolymer containing a polyvinylcaprolactam block and a polyvinylacetate block; a PI3K activator instead of SCF; a TPO receptor agonist instead of TPO; and 4-N-[2-benzyl-7-(2-methyltetrazol-5-yl)-9H-pyrimido[4,5-b]indol-4-yl]cyclohexane-1,4-diamine (UM171). In a certain embodiment of the invention, the culture medium used in the culture method of the invention is an albumin-free, cytokine-free, serum-free medium (preferably a chemically defined medium) that may contain: polyethylene glycol modified with a copolymer containing a polyvinylcaprolactam block and a polyvinylacetate block; a PI3K activator; a TPO receptor agonist; and UM171. As used herein, the wording "the culture medium contains B instead of A" means that the culture medium does not include A, but the culture medium contains B.

In a certain embodiment of the invention, the medium for culturing human hematopoietic stem cells or human blood cells comprises
polyethylene glycol modified with a copolymer containing a polyvinylcaprolactam block and a polyvinylacetate block and
(1) a PI3K activator and at least one compound selected from the group consisting of TPO and TPO receptor agonists;
(2) a TPO receptor agonist and at least one compound selected from the group consisting of stem cell factor (SCF) and PI3K activators; or
(3) a PI3K activator and a TPO receptor agonist.

In this embodiment, the culture medium may be albumin-free. The above medium for culturing human hematopoietic stem cells or human blood cells may further comprise UM171. The above medium for culturing human hematopoietic stem cells or human blood cells may be used in the culture method of the invention. The medium for culturing human hematopoietic stem cells or human blood cells in the invention can be used to expand human hematopoietic stem cells or human blood cells. The medium for culturing human hematopoietic stem cells or human blood cells in the invention can be used to maintain stemness of human hematopoietic stem cells. The medium for culturing human hematopoietic stem cells or human blood cells in the invention can be used to expand human hematopoietic stem cells while maintaining their stemness.

A culture medium suitable for culturing human hematopoietic stem cells or human blood cells can be used, if appropriate, as the culture medium. Examples of a basal medium that can be used include S-clone SF-3 medium, F12 medium, StemSpan (Stem Cell technologies), STEMα (STEM ALPHA), StemPro-34 serum-free medium ( Gibco Invitrogen), StemPro MSC serum-free medium (Invitrogen), HSC-CFU medium (Miltenyl Biotech), S-Clone serum-free medium (SF-02, SF-03, CM-B, SF-B) (Sanko Junyaku), HPGM medium (Sanko Junyaku), AIM V medium (Invitrogen), Marrow MAX bone marrow medium (Invitrogen), KnockOut DMEM /F-12 medium (Invitrogen), Stemline hematopoietic stem cell growth medium (Sigma), SYN serum-free medium (SYN H, SYN B) (AbCys SA), SPE IV medium (AbCys SA), MyeloCult medium (StemCell Technologies), HPG serum-free medium (Lonza), UltraCULTURE medium (Lonza), Opti-MEM medium (Gibco Invitrogen and others), MEM medium (Gibco Invitrogen and others), MEMα (Gibco Invitrogen and others), DMEM medium (Gibco Invitrogen and others), IMDM medium (Gibco Invitrogen and others), PRMI1640 medium (Gibco Invitrogen and others), Ham F-12 medium (Gibco and others),or RD medium. The culture medium includes the basal medium. The culture medium may contain one or more or all of the following, for example, insulin, transferrin (apo), sodium selenite, and/or ethanolamine. The culture medium may contain HEPES, sodium pyruvate, vitamins, amino acids, heparin, heparan sulfate, and/or chondroitin sulfate. The culture medium may contain antibiotics (e.g., penicillin and streptomycin). The culture medium may contain glutamine. The culture medium may contain, for example, insulin, transferrin (apo), sodium selenite, ethanolamine, and antibiotics and may further contain HEPES.

The culture medium in the invention may comprise polyalkylene glycol (e.g., polyethylene glycol) modified with a copolymer containing a polyvinylcaprolactam block and a polyvinylacetate block.

In all the embodiments of the invention, the polyethylene glycol modified with a copolymer containing a polyvinylcaprolactam block and a polyvinylacetate block may be represented by the following chemical formula: 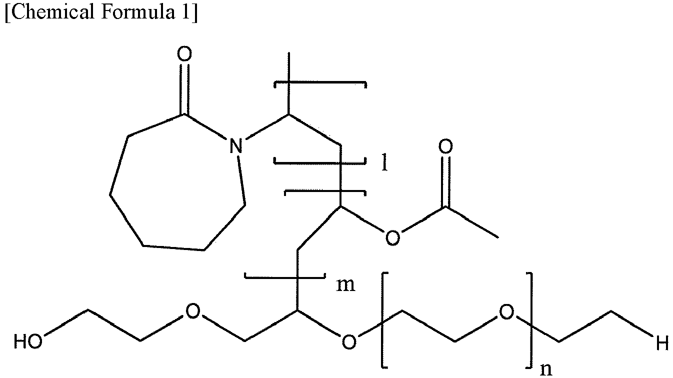 wherein n is any natural number of 5 to 50, m is any natural number of 10 to 100, and 1 is any natural number of 10 to 200.

In a certain embodiment, n may be any natural number from 5 to 20 or 10 to 20, m may be any natural number from 20 to 50 or 30 to 40, and 1 may be any natural number from 30 to 100 or 50 to 60.

The modified polyalkylene glycol may be a compound in which the ethylene glycol unit is an alkylene glycol unit. The alkylene may be a C₁₋₁₀, for example, C₁₋₅, and preferably C₂ or C₃ alkylene.

The culture medium in the invention is designed to expand human hematopoietic stem cells or human hematopoietic cells in the presence of polyethylene glycol modified with a copolymer containing a polyvinylcaprolactam block and a polyvinylacetate block, and may comprise an effective amount of non-cytotoxic PI3K activator and an effective amount of polyalkylene glycol (e.g., polyethylene glycol) modified with a copolymer containing a polyvinylcaprolactam block and a polyvinylacetate block. Here, the culture medium may be albumin-free. The culture medium in the invention may further comprise SCF and/or TPO. The culture medium in the invention may contain an effective amount of non-cytotoxic TPO receptor agonist instead of TPO. The culture medium in the invention may contain an effective amount of non-cytotoxic PI3K activator, an effective amount of PVA, an effective amount of TPO and/or non-cytotoxic TPO receptor agonist, and UM171.

According to the invention, the method of culturing human hematopoietic stem cells may be a method of producing megakaryocyte lineage cells from human hematopoietic stem cells. In this embodiment, the culture medium used in the culture method may be a culture medium containing a PI3K activator and a TPO receptor agonist, wherein the PI3K activator is 740Y-P and the TPO receptor agonist is butyzamide. In this particular embodiment, the culture medium may be free of UM171.

In a certain embodiment of the invention, the culture method of the invention may further comprise culturing in the presence of UM171. In a certain embodiment of the invention, the culture medium used in the culture method of the invention may further comprise UM171.

Human hematopoietic stem cells or human blood cells in the invention may be cultured under conditions suitable for growing the human hematopoietic stem cells or human blood cells. In the invention, the method may further comprise isolating the human hematopoietic stem cells or human blood cells from a culture. The human hematopoietic stem cells may be isolated by any method known to those skilled in the art, such as flow cytometry using hematopoietic stem cell markers. The human hematopoietic stem cells may be obtained as human hematopoietic stem cells or may be further differentiated into other cells before use. When the human hematopoietic stem cells are differentiated into other cells, the cells to be differentiated can be cultured under conditions suitable for the differentiation of these other cells. The human blood cells may also be isolated by any method known to those skilled in the art, such as flow cytometry using markers for the human blood cells.

The invention provides human hematopoietic stem cells or human blood cells obtained by the culture method of the invention. The human hematopoietic stem cells obtained by the culture method of the invention may be purified using CD34 and preferably CD38 as markers. The human hematopoietic stem cells obtained by the culture method of the invention may have a better taking rate after transplantation into a recipient than human hematopoietic stem cells obtained by conventional methods. Thus, the invention provides human hematopoietic stem cells obtained by the culture method of the invention and having a better taking rate after transplantation into a recipient than those before the culture.

In the culture method of the invention, the culturing may be performed in the presence of fibronectin. The culture method of the invention can be performed under conditions allowing for contact between hematopoietic stem cells and fibronectin. The culturing is preferably carried out in the culture method of the invention, for instance, such that the inside (e.g., the bottom) of cultureware is coated with fibronectin.

The albumin-free medium used in the culture method of the invention contains less than 0.1 (w/v)%, 0.05 (w/v)%, 0.01 (w/v)%, 0.005 (w/v)%, 0.001 (w/v)%, 0.0005 (w/v)%, or 0.0001 (w/v)% serum albumin, or is completely free of albumin.

The culture medium used in the culture method of the invention may contain recombinant TPO. The recombinant TPO may be, for example, mammalian recombinant TPO, or recombinant human TPO. In a certain embodiment of the invention, the TPO concentration is preferably from 20 to 200 ng/mL, more preferably from 30 to 150ng/mL, and still more preferably from 40 to 150ng/mL, and may be, for example, 100 ng/mL.

The culture medium used in the culture method of the invention may further contain recombinant SCF. The recombinant SCF may be, for example, mammalian recombinant SCF, or recombinant human SCF. In a certain embodiment of the invention, the SCF concentration is preferably from 1 to 200 ng/mL, more preferably from 1 to 150 ng/mL, still more preferably from 1 to 100 ng/mL (e.g., from 1 to 50 ng/mL), still more preferably from 1 to 30 ng/mL, and still more preferably from 1 to 20 ng/mL (e.g., from 5 to 15 ng/mL).

The culture medium used in the culture method of the invention may contain recombinant TPO and recombinant SCF. In this embodiment, the TPO concentration is preferably from 20 to 200 ng/mL, more preferably from 30 to 150 ng/mL, still more preferably from 40 to 150 ng/mL (e.g., 100 ng/mL) and the SCF concentration is preferably from 1 to 200 ng/mL, more preferably from 1 to 150 ng/mL, still more preferably from 1 to 100 ng/mL (e.g., from 1 to 50 ng/mL), still more preferably from 1 to 30 ng/mL, and still more preferably from 1 to 20 ng/mL (e.g., from 5 to 15 ng/mL). In a preferred embodiment, the medium used in the culture method pf the invention may contain 40 to 150 ng/mL recombinant TPO and 1 to 50 ng/mL recombinant SCF. In a preferred embodiment, the medium used in the culture method pf the invention may have a TPO concentration higher than the SCF concentration, and the TPO concentration may be, for example, any of the concentrations in the above concentration range, and may be 2-, 3-, 4-, 5-, 6-, 7-, 8-, 9-, or 10-fold or higher.

The culture medium used in the culture method of the invention may further contain FLT3L (in particular, human FLT3L). FLT3 is a cell surface receptor (also called CD135) known as FMS-related tyrosine kinase 3. FLT3 is expressed on the surface of, for instance, each hematopoietic stem cell. FLT3L is a ligand for FLT3 and participates in normal development of hematopoietic stem cells and hematopoietic progenitors. The culture medium used in the culture method of the invention may also contain, in addition to FLT3L, IL-3 and/or GM-CSF. Interleukin-3 (IL-3) is a hematopoietic growth factor and plays an important role in the proliferation and survival of myeloid progenitor cells. GM-CSF is granulocyte-monocyte colony-stimulating factor and a cytokine that promotes differentiation into hematopoietic stem cells. GM-CSF, acting in concert with IL-3 and IL-5, can induce hematopoietic stem cells to differentiate into myeloid progenitor cells. For example, GM-CSF can induce hematopoietic stem cells to differentiate into, for example, burst-forming unit-erythroids (BFU-E), granulocyte-monocyte colony-forming units (CFU-GM), eosinophil colony-forming units (CFU-Eo), and basophil colony-forming units (CFU-Ba). GM-CSF is responsible for the differentiation of CFU-GM into neutrophils and monocytes, and CFU-Eo into eosinophils. CFU-Ba can be differentiated into eosinophils by IL-3 or IL-5. Thus, the culture medium used in the culture method of the invention may contain FLT3L and may further contain IL-3 and/or GM-CSF. In addition, the culture method of the invention can be implemented under conditions suitable for the proliferation and/or differentiation of each blood cell. Such culture conditions are well-known as culture conditions for an albumin-containing medium, and are likewise applicable to the culture method of the invention using a medium with reduced albumin or without any albumin.

The culture medium used in the culture method of the invention may contain a PI3K activator instead of SCF and/or a TPO receptor agonist instead of TPO. The culture medium used in the culture method of the invention may contain a PI3K activator instead of SCF and a TPO receptor agonist instead of TPO. The culture medium used in the culture method of the invention may be a cytokine-free medium that contains a PI3K activator and a TPO receptor agonist. The culture medium used in the culture method of the invention may contain a PI3K activator instead of SCF and a TPO receptor agonist instead of TPO as well as may further contain UM171. The culture medium used in the culture method of the invention may be a cytokine-free medium that contains a PI3K activator, a TPO receptor agonist, and UM171.

The method of culturing human hematopoietic stem cells according to the invention may further comprise causing hematopoietic stem cells to proliferate under conditions sufficient for maintenance and/or proliferation of the hematopoietic stem cells. In this embodiment, for example, hematopoietic stem cells are made to expand 10-, 50-, 100-, 200-, 300-, 400-, or 500-fold or more from the start of culture.

The conditions sufficient for maintenance and/or proliferation of the human hematopoietic stem cells may be, for example, conditions in which they are cultured in the above culture medium. The conditions sufficient for maintenance and/or proliferation of the human hematopoietic stem cells may preferably be, for example, conditions in the presence of fibronectin or may be conditions in which the human hematopoietic stem cells can be in contact with fibronectin.

The method of culturing human blood cells according to the invention may further comprise causing human blood cells to proliferate under conditions sufficient for maintenance and/or proliferation of the human blood cells. In this embodiment, for example, hematopoietic stem cells are made to expand 10-, 50-, 100-, 200-, 300-, 400-, or 500-fold or more from the start of culture.

The culture method of the invention may further comprise
collecting the expanded human hematopoietic stem cells or human blood cells from the culture medium.

The collected human hematopoietic stem cells or human blood cells may be further enriched or isolated. The human hematopoietic stem cells or human blood cells may be enriched or isolated using a cell surface marker(s). Examples of the cell surface marker(s) that can be used for the enrichment or isolation of human hematopoietic stem cells include CD34 and/or CD38. The hematopoietic stem cells or human blood cells may be enriched or isolated using a cell sorter.

Another embodiment of the invention provides a method of *ex vivo* producing human hematopoietic stem cells or human blood cells, comprising: the culture method of the invention. The production method of the invention makes it possible to produce functional human hematopoietic stem cells or human blood cells.

As used herein, the term "functional" means that a hematopoietic system can be restored in a human individual (recipient) in which human hematopoietic stem cells have been transplanted.

### Examples

Reference Example 1: Test for Growth of Hematopoietic Stem Cells by Using Albumin-Free Culture Medium

In this Reference Example, mouse hematopoietic stem cells (KSL) and human hematopoietic stem cells (CD34⁺CD38⁻) were tested for growth using an albumin-free culture medium. The culture medium was a serum-free medium containing polyvinyl alcohol (PVA) and free of albumin as described in Wilkinson et al., Nature, 571: 117-121, 2019.

Specifically, for culturing mouse hematopoietic stem cells, the culture medium was F12 medium containing 1% insulin-transferrin-selenium-ethanolamine (ITSX), 10 mM HEPES, 1% penicillin-streptomycin-glutamine, 100 ng/mL mouse thrombopoietin (mTPO), and 10 ng/mL mouse stem cell factor (mSCF). The above medium contained PVA at a final concentration of 0.1%. For culturing human hematopoietic stem cells, the culture medium was IMDM medium containing 1% insulin-transferrin-selenium-ethanolamine (ITSX), 25 mM HEPES, 1% penicillin-streptomycin-glutamine, 100 ng/mL human thrombopoietin, and 10 ng/mL human stem cell factor (hSCF). The above medium contained PVA at a final concentration of 0.1%.

In all the media used in the following Examples, IMDM medium (hereinafter, also referred to as "common medium") containing 1% insulin-transferrin-selenium-ethanolamine (ITSX), 25 mM HEPES, 1% penicillin-streptomycin-glutamine, and PVA was used unless otherwise indicated. Thus, hereinafter, we will focus on the components added to the common medium and describe the medium. For example, since the above medium contains TPO and SCF in addition to the common medium, the above medium is called TPO+SCF medium for convenience. When the concentration of each factor is also expressed, it may be written as SCF10+TPO100 or abbreviated as S10+T100.

KSL cells from mouse bone marrow were used as mouse hematopoietic stem cells. Specifically, the mouse bone marrow cells were isolated from the tibia, femur, and pelvis, and stained with an APC-c-KIT antibody. The c-KIT⁺ cells were enriched using anti-APC magnetic beads and LS column (Miltenyi Biotec). The c-KIT-enriched cells were then stained with a lineage antibody cocktail (biotinylated CD4, CD8, CD45R, TER119, LY-6G/LY-6C, and CD127) before stained with an anti-CD34 antibody, an anti-cKIT antibody, an anti-SCAl antibody, and streptavidin-APC/eFluor 780 for 90 min. Cell populations were then purified with a FACS Aria II (BD) by direct sorting into wells containing a medium while using propidium iodide as a death staining marker.

Human hematopoietic stem cells used were CD34⁺CD38⁻ cells from human bone marrow. Specifically, commercially available human bone marrow CD34-positive cells (Lonza 2C-101) were purchased.

The results are shown in FIG. 1. According to Wilkinson *et al*., 2019, mouse hematopoietic stem cells failed to maintain long-term proliferation in an albumin-free, serum-free medium even in the presence of SCF and TPO. However, Wilkinson *et al*., 2019, showed that addition of PVA to the medium permits mouse hematopoietic stem cells to proliferate for a long period of time in an albumin-free, serum-free medium. As shown and observed in FIG. 1, mouse hematopoietic stem cells proliferated well in a PVA-containing, albumin-free, serum-free medium, but human hematopoietic stem cells did not proliferate well in this medium.

### Reference Example 2: Differences in Signaling between Mouse and Human Hematopoietic Stem Cells

Signaling pathways of mouse and human hematopoietic stem cells in the presence of SCF and TPO were analyzed.

Phosphorylation immunostaining was used to analyze the degree of phosphorylation of each signaling factor downstream of SCF and TPO in mouse or human hematopoietic stem cells in the presence of 10 ng/mL stem cell factor (SCF) and 100 ng/mL thrombopoietin (TPO), in the absence of albumin, and in the presence of PVA. First, how much each factor downstream of SCF and TPO signaling was phosphorylated was detected using an antibody specific for each phosphorylation form of the factor. Specifically, anti-phosphorylation antibodies against representative signaling molecules such as Akt, PI3K, and Stat5 were used and reacted with cells stimulated with cytokines, and the fluorescence intensity of each antibody reacting with the cells was quantitatively analyzed by fluorescence microscopy.

As shown in FIG. 2, among the seven factors examined, several factors were found to have different phosphorylation statuses between mouse and human hematopoietic stem cells. Among them, for PI3K and Akt, the phosphorylated forms of PI3K and Akt were highly required in mouse hematopoietic stem cells 24 h after the addition of SCF and TPO, whereas human hematopoietic stem cells had little or no phosphorylated forms or less than the levels of phosphorylated forms in mouse hematopoietic stem cells.

Then, human hematopoietic stem cells were cultured in a culture medium supplemented with 0.3 µM AKTa (Sigma-Aldrich, product No. 123871) or 20 µM PI3Ka. Cells were counted after 3, 5, and 7 days of incubation, and the relative cell count at each time point was calculated while the cell count on the first day of incubation was set to 1. In the following Examples, 740Y-P (Provider name: Tocris, product No. 1983) was used as PI3Ka. As shown in FIG. 3, human hematopoietic stem cells showed marked proliferation in the presence of PI3Ka. By contrast, no proliferative effect of AKTa on human hematopoietic stem cells was observed under the conditions of this experiment.

Next, human hematopoietic stem cells were cultured to examine whether PI3Ka could completely replace SCF. Human hematopoietic stem cells were cultured for 7 days in the above culture medium for human hematopoietic stem cells with 20 µM PI3Ka (S10+PI3Ka20+T100) or in the above culture medium with 20 µM PI3Ka but without SCF (PI3Ka20+T100). The total number of cells and the number of isolated CD34⁺ cells as obtained by cell sorting using an anti-CD34 antibody were then determined. The results are shown in FIG. 4. As shown in FIG. 4, when PI3Ka was added, there was no significant difference in the number of total cells or CD34⁺ cells with or without SCF.

Next, human hematopoietic stem cells were cultured to examine whether TPO could be replaced by a TPO receptor agonist. Examples of the known TPO receptor agonist include butyzamide, eltrombopag, or avatrombopag. Human hematopoietic stem cells were cultured in a medium containing 0.1 µM butyzamide, 3 µg/mL eltrombopag, or 3 µM avatrombopag in the presence of 0.1% recombinant human serum albumin (Albumin Biosciences) or in the presence of PVA in the above culture medium without TPO. In this experiment, Mpl32D cells were used as human hematopoietic stem cells. The results are shown in FIG. 5. As shown in FIG. 5, in the presence of albumin, butyzamide, eltrombopag, or avatrombopag was able to support proliferation of human hematopoietic stem cells in the absence of TPO. In contrast, in the presence of PVA (in the absence of albumin), butyzamide produced a marked cell proliferative effect on human hematopoietic stem cells in the absence of TPO, whereas the effect was small for avatrombopag and almost none for eltrombopag.

In addition, purchased human bone marrow CD34⁺ cells (provider name: Lonza, product No. 2C-101) or CD34⁺ cells isolated using microbeads from given fresh cord blood were used for culture experiments. the experiments were performed in 24-well plates at 0.2 to 1.0 × 10⁵ cells per well, with TPO removed from the culture medium for human hematopoietic stem cells and replaced by one of the above TPO receptor agonists (hereinafter, sometimes referred to as "TPOago"). The cell count on the seventh day of culture relative to the cell count on the first day of culture was determined. The results are shown in FIG. 6. As shown in FIG. 6, human hematopoietic stem cells exhibited significant cell proliferation only in the presence of butyzamide in the absence of albumin and in the presence of PVA. In the absence of albumin and in the presence of PVA, the presence of avatrombopag or eltrombopag caused cell death in hematopoietic stem cells. However, in the *in vivo* setting, these TPO receptor agonists are safe compounds used to treat surgically treated patients with cirrhosis or patients with aplastic anemia. The results of this Example indicate that some TPO receptor agonists can be cytotoxic to human hematopoietic stem cells in the absence of albumin and in the presence of PVA. This also suggests that some TPO receptor agonists are cytotoxic to human hematopoietic stem cells, and that use of each agonist that is not cytotoxic to human hematopoietic stem cells is sufficient for culturing human hematopoietic stem cells.

Next, it was examined whether or not SCF and TPO could be replaced by PI3Ka and a TPO receptor agonist in the absence of albumin and in the presence of PVA. In the following Examples, butyzamide was used as a TPO receptor agonist. In addition, purchased human bone marrow CD34⁺ cells (provider name: Lonza, product No. 2C-101) or CD34⁺ cells isolated using microbeads from given fresh cord blood as described above were used for culture experiments. First, 0.2 to 1.0 × 10⁵ cells per well of a 24-well plate were dispensed, and were cultured in a medium with a composition in which SCF was replaced by 20 µM PI3Ka (PI3Ka20µM+TPO100) and in a medium with a composition where SCF was replaced by 20 µM PI3Ka and TPO was replaced by 0.1 µM butyzamide (PI3Ka20µM+TPOago0.1µM), respectively. After 7 days, the total number of cells was determined and the number of CD34⁺ cells was determined by flow cytometry. Thereafter, the growth rate compared to that at the beginning of the incubation was determined. The results are shown in FIG. 7. FIG. 7 clearly demonstrates that butyzamide can completely replace TPO. In addition, CD34⁺ cells were sorted before and after incubation by using a cell sorter, and 100 cells were seeded into Methocult H4415 for colony assay. After two weeks, each colony was picked under a microscope, a cytospin sample was prepared, and the sample was subjected to Giemsa staining. The type of the colony was then identified under a microscope. GEmM colonies per 50 CD34⁺ cells were counted and a rate of increase in the number of colonies compared to before the culture was determined. It has then been found that butyzamide can completely replace TPO with respect to GEmM colony formation potential.

Next, human hematopoietic stem cells were cultured in a medium containing 20 µM PI3Ka and/or 0.1 µM TPOago with reference to a culture medium without SCF or TPO. On day 7 of culture, the number of total cells and the number of CD34⁺ cells included were counted by flow cytometry, and the growth rate relative to that at the start of culture was determined. As shown in FIG. 8, PI3Ka alone or butyzamide alone was found to cause no increase in the cell count, but the presence of both PI3Ka and TPOago was found to cause a marked increase in the cell count.

Further, the next step was to determine which cell population was more likely to proliferate in a medium in which SCF and TPO were replaced by PI3Ka and TPOago. Given fresh cord blood was used to isolate CD34⁺ cells by using microbeads as described above, and the cells were used for culture experiments. The cells were then fractionated with a cell sorter using an anti-CD34-PE-Cy7 antibody (provider name BD Biosciences, product No. 348791), an anti-CD38-V450 antibody (provider name BD Biosciences, product No. 646851), an anti-CD133-PE antibody (provider name Miltenyi Biotec, product No. 130-080-801), an anti-CD45RA-APC antibody (provider name BioLegend, product No. 304112), and an anti-CD49f-PE antibody (provider name BioLegend, product No. 313611). For each of the CD34⁺ fraction, CD34⁺CD38⁻CD133⁺ fraction, or CD34⁺CD38⁻CD45RA⁻CD49f⁺ fraction, which molecules have been reported as purification markers for human hematopoietic stem cells derived from human cord blood, the total cell count and the number of CD34⁺ cells included were counted by flow cytometry on day 7 after the start of culture, and the growth rate relative to that at the start of culture was determined. The results are shown in FIG. 9. As shown in FIG. 9, significant cell proliferation was observed in all cell fractions, but the CD34⁺CD38⁻CD133⁺ fraction and the CD34⁺CD38⁻CD45RA⁻CD49f⁺ fraction, especially the CD34⁺CD38⁻CD45RA⁻CD49f⁺ fraction exhibited marked cell growth.

### Reference Example 3: Experiment for Long-Term Culture of Human Hematopoietic Stem Cells

Human hematopoietic stem cells were cultured in the above culture medium for human hematopoietic stem cells, which medium contained 20 µM PI3Ka and 0.1 µM TPOago instead of SCF and TPO. The total cell count and the CD34⁺ cell count were determined as above on days 7 and 14 after the start of culture. The results are shown in FIG. 10. As shown in FIG. 10, the total cell count increased with the number of days of incubation, while the CD34⁺ cell count decreased at day 14 compared to day 7.

When an optical microscope was used to observe cultured cells, giant cells were detected on day 14. To check the possibility that these giant cells were megakaryocytes or megakaryocyte progenitors, the cells were fractionated by flow cytometry using an anti-CD41a-FITC antibody (provider name BD Pharmingen, product No. 555466) and an anti-CD42b antibody (provider name BD Pharmingen, product No. 555473). As shown in FIG. 11, the results showed that the majority of cells at 14 days after the start of culture were CD41a⁺CD42b⁺ cells. However, as shown in FIG. 11, CD34⁺CD38⁻ cells were still proliferating under these conditions.

In addition, CD34⁺ cells were obtained from the culture at day 14 after the start of culture, and 100 cells were seeded into Methocult H4415 for colony assay. After 2 weeks, colonies were collected and cytospin specimens were prepared. The specimens were then subjected to Giemsa staining, and the colony types were determined under a microscope. The results are shown in FIG. 12. Regarding the colony types, G denotes "granulocytes"-, E denotes "erythroblasts"-, m denotes "macrophages"-, and M denotes "megakaryocytes"-containing colonies. As shown in FIG. 12, most of the cell colonies contained megakaryocytes.

### Reference Example 4: Further Investigation of Conditions Suitable for Long-Term Culture of Human Hematopoietic Stem Cells

Long-term culture of human hematopoietic stem cells was attempted in the presence of compounds capable of growing human hematopoietic stem cells.

Prepared was the above culture medium for human hematopoietic stem cells, which medium contained 20 µM PI3Ka and 0.1 µM TPOago instead of SCF and TPO (PI3Ka20µM+TPOago0.1µM), or which medium contained SR-1 (500nM) and/or UM171 (35 nM) (+SR-1, +UM171, or +SR-1+UM171). In addition, purchased human bone marrow CD34⁺ cells (provider name: Lonza, product No. 2C-101) or CD34⁺ cells isolated using microbeads from given fresh cord blood as described above were used for culture experiments. First, 0.2 to 1.0 × 10⁵ cells per well of a 24-well plate were dispensed, and the CD34⁺ cells were cultured in the prepared medium. The total cell count and the CD34⁺ cell count were determined as above on day 14 after the start of culture, and the growth rate relative to that at the start of culture was determined. The results are shown in FIG. 13. As shown in FIG. 13, the total cell count and the CD34⁺ cell count were increased in the culture medium further containing UM171, and the growth rate of CD34⁺ cells was significantly larger in the medium containing UM171 than in the medium without UM171. In contrast, SR-1 caused cell death under these experimental conditions.

Further, the growth rate of the total cell count or the CD34⁺ cell count was determined by culturing the CD34⁺ cells under three conditions: condition 1: cultured for 14 days in the culture medium for human hematopoietic stem cells above, which medium contained 20 µM PI3Ka and 0.1 µM TPOago instead of SCF and TPO (PI3Ka20µM+TPOago0.1µM), condition 2: at day 7 or later, cultured in a medium without butyzamide (Day7-no Buty), and condition 3: cultured for 14 days in a medium (PI3Ka20µM+TPOago0.1µM) further containing UM171 (+UM171). The CD41⁺ cell count was determined using a flow cytometer. The results are shown in FIG. 14. As shown in FIG. 14, the CD34⁺ cell count increased and the CD41⁺ cell count decreased under condition 2, in which the cells were cultured in the medium without butyzamide on day 7 or later after the start of culture, when compared to condition 1. As shown in FIG. 14, under condition 3, in which the cells were cultured in the medium further containing UM171, the growth rate of CD34⁺ cells was markedly increased and the CD41⁺ cell count was markedly decreased compared to condition 1 or 2. Thus, under serum-free medium conditions in the presence of PVA, PI3Ka and TPOago, UM171 was found to cause human hematopoietic stem cells to proliferate and to inhibit their differentiation into megakaryocyte progenitors and megakaryocytes (see FIG. 15).

### Reference Example 5: Experiment for Transplantation of CD34⁺ Cells after Culture

Human CD34⁺ cells cultured for 7 days under each of conditions 1 to 3 were transplanted into irradiated NOG mice to check cell engraftment. Specifically, 1 × 10⁴ cultured human CD34+ cells were transplanted into each NOG mouse irradiated with 1.5 Gy of γ-rays. Twelve weeks after transplantation, peripheral blood of each NOG mouse was collected, and the cellular components in the peripheral blood were analyzed using a flow cytometer. The results are shown in FIG. 16. As shown in FIG. 16, in conditions 1 and 2, HSC (hematopoietic stem cell) engraftment rates increased from 14.9% and 11.1%, when pre-cultured CD34⁺ cells were engrafted, to 66.6% and 54.9%, respectively, when post-cultured CD34+ cells were engrafted. In condition 3, the HSC engraftment rate increased from 17%, when pre-cultured CD34⁺ cells were engrafted, to 67%, when post-cultured CD34⁺ cells were engrafted.

These results have revealed that the PI3K activator and the TPO receptor agonist can induce favorable proliferation of human hematopoietic stem cells under serum-free medium conditions in the absence of albumin and in the presence of PVA, and that the proliferated human CD34⁺ cells can maintain their HSC characteristics and the engraftment rate after transplantation into other individuals can be improved. In addition, human hematopoietic stem cells tend to proliferate as CD34⁺ cells, while some of them differentiate into megakaryocytes when cultured under the conditions for a long period, thereby producing megakaryocytes. The addition of UM171 to the cells increased the growth rate of CD34⁺ cells and inhibited their differentiation into megakaryocytes.

Next, human mononuclear cells were isolated from fresh human cord blood. Under conditions 1 and 3, the obtained cells (5 × 10⁵ cells) were cultured, and the total cell count, the CD34⁺ cell count, the percentage of CD34⁺ cells in the total cells, and the cell viability were each determined on day 7 and day 14 after the start of culture. The results are shown in FIG. 17. As shown in FIG. 17, under any of the conditions, the total cell count of mononuclear cells obtained from human cord blood decreased with incubation. On the other hand, under condition 3, the percentage of CD34⁺ cells in the total cells was markedly larger than under condition 1. The cell viability was also significantly higher in condition 3 than in condition 1. Further, the CD34⁺ cell count showed a marked increase in condition 3, while the cell count was maintained favorably in condition 1 whereas no increase in the cell count was observed.

### Example 1: Test for Growth of Hematopoietic Stem Cells by Using Albumin-Free Culture Medium

In this Example, mouse hematopoietic stem cells (KSL) and human hematopoietic stem cells (CD34⁺CD38⁻) were tested for growth using an albumin-free culture medium. The culture medium was an albumin-free, serum-free medium, as described in Wilkinson et al., Nature, 571: 117-121, 2019, that contained each kind of polymer instead of polyvinyl alcohol (PVA).

Specifically, for culturing mouse hematopoietic stem cells, the culture medium was Ham's F12 medium containing 1% insulin-transferrin-selenium-ethanolamine (ITSX), 10 mM HEPES, 1% penicillin-streptomycin-glutamine, 100 ng/mL mouse thrombopoietin (mTPO), and 10 ng/mL mouse stem cell factor (mSCF).

For culturing human hematopoietic stem cells, the culture medium was IMDM medium containing 1% insulin-transferrin-selenium-ethanolamine (ITSX), 25 mM HEPES, 1% penicillin-streptomycin-glutamine, 100 ng/mL human thrombopoietin (hTPO), and 10 ng/mL human stem cell factor (hSCF). In the following Examples, unless otherwise indicated, the above culture medium was used to culture hematopoietic stem cells.

The polymer added to the medium was as follows: polyvinylpyrrolidone K12, povidone K17, povidone, polyoxyethylene polyoxypropylene glycol, a polyvinylcaprolactam-polyvinylacetate-polyethylene glycol graft copolymer (hereinafter referred to as "polymer A"). More specifically, prepared was each culture medium containing, at a final concentration of 0.1%, BASF's Kolliphor (trademark) P188 bio (188 bio), Kolliphor (trademark) P 188 Geismar (188 Geismar), Soluplus (Sol+), Kolliphor (trademark) P 407 Geismar (407 Geismar), Kollidon (trademark) 30 Origin USA (30 USA), Kollidon (trademark) 17 PF (17 PF), Kollidon (trademark) 25 (25), Kollidon (trademark) 90F (90F), or Kollidon (trademark) 12PF (12PF)

A KSL cell fraction from mouse bone marrow CD34⁻CD150⁺ cells was used as mouse hematopoietic stem cells. Specifically, the mouse bone marrow cells were isolated from the tibia, femur, and pelvis, and stained with an APC-c-KIT antibody. The c-KIT⁺ cells were enriched using anti-APC magnetic beads and LS column (Miltenyi Biotec). The c-KIT-enriched cells were then stained with a lineage antibody cocktail (biotinylated CD4, CD8, CD45R, TER119, LY-6G/LY-6C, and CD127) before stained with an anti-CD34 antibody, an anti-cKIT antibody, an anti-SCAl antibody, and streptavidin-APC/eFluor 780 for 90 min. Cell populations were then purified with a FACS Aria II (BD) by direct sorting into wells containing a medium while using propidium iodide as a death staining marker.

Human hematopoietic stem cells used were CD34⁺CD38⁻ cells from human bone marrow. Specifically, commercially available human bone marrow CD34-positive cells (Lonza 2C-101) were purchased.

Mouse hematopoietic stem cells were cultured in each medium for one week, and the number of cells after culture was counted.

The results are shown in FIG. 18. As shown in FIG. 18, mouse hematopoietic stem cells proliferated well in the albumin-free, serum-free medium containing PVA, but not in the presence of any of other compounds tested in place of PVA, and only in the presence of compound A.

### Soluplus (trademark) is a compound with the following structure:

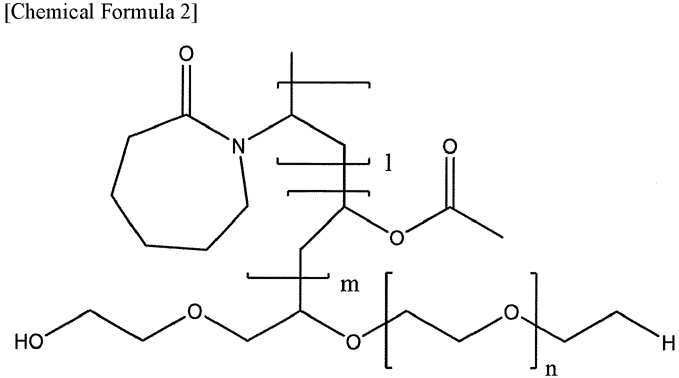

wherein n is 13, m is 30, and 1 is 57.

Note that according to Wilkinson *et al*., 2019, mouse hematopoietic stem cells failed to maintain long-term proliferation in an albumin-free, serum-free medium even in the presence of SCF and TPO. However, Wilkinson *et al*., 2019, shows in Figure 2b that addition of PVA to the medium permits mouse hematopoietic stem cells to proliferate for a long period of time in an albumin-free, serum-free medium.

This has revealed that mouse hematopoietic stem cells proliferate well in the presence of PVA or polymer A, even in an albumin-free, serum-free medium.

Mouse hematopoietic stem cells were dispensed at 50 cells/well and cultured in the presence of 0.1% PVA or polymer A and SCF and TPO. The results are shown in FIG. 19. FIG. 19 has revealed that mouse hematopoietic stem cells well-formed each colony in the presence of PVA or polymer A, even in an albumin-free, serum-free medium. This has indicated that hematopoietic stem cells have a mitotic potential in the presence of PVA or polymer A.

The percentage of CD201-positive cells among the proliferated cells was checked using an alkaline phosphatase-conjugated anti-mouse CD201 antibody (eBio1560). Then, as shown in FIG.20, mouse hematopoietic stem cells cultured in the presence of polymer A had a greater percentage of CD201-positive cells than in the presence of PVA. CD201 is a known marker of hematopoietic stem cells, and the high percentage of CD201-positive cells suggests that stemness was better maintained after proliferation.

Further, mouse hematopoietic stem cells were cultured in the presence of polymer A or PVA at a final concentration of 0.1% for 1 week and then transplanted into irradiated mice. Four weeks after the transplantation, the peripheral blood of each irradiated mouse was analyzed to determine the chimerism rate of blood cells derived from the transplanted hematopoietic stem cells. As a result, the hematopoietic stem cells cultured in the presence of polymer A showed a chimerism rate (contribution rate) comparable to that of hematopoietic stem cells cultured in PVA. This indicates that the bone marrow reconstruction potential of hematopoietic stem cells cultured in the presence of polymer A was equivalent to that of hematopoietic stem cells cultured in the presence of PVA.

### Example 2: Differences in Signaling between Mouse and Human Hematopoietic Stem Cells

Signaling pathways of mouse and human hematopoietic stem cells in the presence of SCF and TPO were analyzed.

Phosphorylation immunostaining was used to analyze the degree of phosphorylation of each signaling factor downstream of SCF and TPO in mouse or human hematopoietic stem cells in the presence of 10 ng/mL stem cell factor (SCF) and 100 ng/mL thrombopoietin (TPO), in the absence of albumin, and in the presence of PVA. First, how much each factor downstream of SCF and TPO signaling was phosphorylated was detected using an antibody specific for each phosphorylation form of the factor. Specifically, anti-phosphorylation antibodies against representative signaling molecules such as Akt, PI3K, and Stat5 were used and reacted with cells stimulated with cytokines, and the fluorescence intensity of each antibody reacting with the cells was quantitatively analyzed by fluorescence microscopy.

As shown in FIG. 24, among the seven factors examined, several factors were found to have different phosphorylation statuses between mouse and human hematopoietic stem cells. Among them, for PI3K and Akt, the phosphorylated forms of PI3K and Akt were highly required in mouse hematopoietic stem cells 24 h after the addition of SCF and TPO, whereas human hematopoietic stem cells had little or no phosphorylated forms or less than the levels of phosphorylated forms in mouse hematopoietic stem cells.

Then, human hematopoietic stem cells were cultured in a culture medium supplemented with 0.3 µM AKTa (Sigma-Aldrich, product No. 123871) or 20 µM PI3Ka. Cells were counted after 3, 5, and 7 days of incubation, and the relative cell count at each time point was calculated while the cell count on the first day of incubation was set to 1. In the following Examples, 740Y-P (Provider name: Tocris, product No. 1983) was used as PI3Ka. As shown in FIG. 25, human hematopoietic stem cells showed marked proliferation in the presence of PI3Ka. By contrast, no proliferative effect of AKTa on human hematopoietic stem cells was observed under the conditions of this experiment.

Next, human hematopoietic stem cells were cultured to examine whether PI3Ka could completely replace SCF. Human hematopoietic stem cells were cultured for 7 days in the above culture medium for human hematopoietic stem cells with 20 µM PI3Ka (S10+PI3Ka20+T100) or in the above culture medium with 20 µM PI3Ka but without SCF (PI3Ka20+T100). The total number of cells and the number of isolated CD34⁺ cells as obtained by cell sorting using an anti-CD34 antibody were then determined. The results are shown in FIG. 26. As shown in FIG. 26, when PI3Ka was added, there was no significant difference in the total number of cells or CD34⁺ cells with or without SCF.

Next, human hematopoietic stem cells were cultured to examine whether TPO could be replaced by a TPO receptor agonist. Examples of the known TPO receptor agonists include butyzamide, eltrombopag, or avatrombopag. Human hematopoietic stem cells were cultured in a medium containing 0.1 µM butyzamide, 3 µg/mL eltrombopag, or 3 µM avatrombopag in the presence of 0.1% recombinant human serum albumin (Albumin Biosciences) or in the presence of PVA in the above culture medium without TPO. In this experiment, Mpl32D cells were used as human hematopoietic stem cells. The results are shown in FIG. 27. As shown in FIG. 27, in the presence of albumin, butyzamide, eltrombopag, or avatrombopag was able to support proliferation of human hematopoietic stem cells in the absence of TPO. In contrast, in the presence of PVA (in the absence of albumin), butyzamide produced a marked cell proliferative effect on human hematopoietic stem cells in the absence of TPO, whereas the effect was small for avatrombopag and almost none for eltrombopag.

In addition, purchased human bone marrow CD34⁺ cells (provider name: Lonza, product No. 2C-101) or CD34⁺ cells isolated using microbeads from given fresh cord blood were used for culture experiments. Experiments were performed in 24-well plates with 0.2 to 1.0 × 10⁵ cells per well, with TPO removed from the culture medium for human hematopoietic stem cells and replaced by one of the above TPO receptor agonists (hereinafter, sometimes referred to as "TPOago"). The cell count on the seventh day of culture relative to the cell count on the first day of culture was determined. The results are shown in FIG. 28. As shown in FIG. 28, human hematopoietic stem cells exhibited significant cell proliferation only in the presence of butyzamide in the absence of albumin and in the presence of PVA. In the absence of albumin and in the presence of PVA, the presence of avatrombopag or eltrombopag caused cell death in hematopoietic stem cells. However, in the *in vivo* setting, these TPO receptor agonists are safe compounds used to treat surgically treated patients with cirrhosis or patients with aplastic anemia. The results of this Example indicate that some TPO receptor agonists can be cytotoxic to human hematopoietic stem cells in the absence of albumin and in the presence of PVA. This also suggests that some TPO receptor agonists are cytotoxic to human hematopoietic stem cells, and that use of each agonist that is not cytotoxic to human hematopoietic stem cells is sufficient for culturing human hematopoietic stem cells.

Next, it was examined whether or not SCF and TPO could be replaced by PI3Ka and a TPO receptor agonist in the absence of albumin and in the presence of PVA. In the following Examples, butyzamide was used as a TPO receptor agonist. In addition, purchased human bone marrow CD34⁺ cells (provider name: Lonza, product No. 2C-101) or CD34⁺ cells isolated using microbeads from given fresh cord blood as described above were used for culture experiments. First, 0.2 to 1.0 × 10⁵ cells per well of a 24-well plate were dispensed, and were cultured in a medium with a composition in which SCF was replaced by 20 µM PI3Ka (PI3Ka20µM+TPO100) and in a medium with a composition where SCF was replaced by 20 µM PI3Ka and TPO was replaced by 0.1 µM butyzamide (PI3Ka20µM+TPOago0.1µM), respectively. After 7 days, the total number of cells was determined and the number of CD34⁺ cells was determined by flow cytometry. Thereafter, the growth rate compared to that at the beginning of the incubation was determined. The results are shown in FIG. 29. FIG. 29 clearly demonstrates that butyzamide can completely replace TPO. In addition, CD34⁺ cells were sorted before and after incubation by using a cell sorter, and 100 cells were seeded into Methocult H4415 for colony assay. After two weeks, each colony was picked under a microscope, a cytospin sample was prepared, and the sample was subjected to Giemsa staining. The type of the colony was then identified under a microscope. GEmM colonies per 50 CD34⁺ cells were counted and a rate of increase in the number of colonies compared to before the culture was determined. It has then been found that butyzamide can completely replace TPO with respect to GEmM colony formation potential.

Next, human hematopoietic stem cells were cultured in a medium containing 20 µM PI3Ka and/or 0.1 µM TPOago with reference to a culture medium without SCF or TPO. On day 7 of culture, the number of total cells and the number of CD34⁺ cells included were counted by flow cytometry, and the growth rate relative to that at the start of culture was determined. As shown in FIG. 30, PI3Ka alone or butyzamide alone was found to cause no increase in the cell count, but the presence of both PI3Ka and TPOago was found to cause a marked increase in the cell count.

Further, the next step was to determine which cell population was more likely to proliferate in a medium in which SCF and TPO were replaced by PI3Ka and TPOago. Given fresh cord blood was used to isolate CD34⁺ cells by using microbeads as described above, and the cells were used for culture experiments. The cells were then fractionated with a cell sorter using an anti-CD34-PE-Cy7 antibody (provider name BD Biosciences, product No. 348791), an anti-CD38-V450 antibody (provider name BD Biosciences, product No. 646851), an anti-CD133-PE antibody (provider name Miltenyi Biotec, product No. 130-080-801), an anti-CD45RA-APC antibody (provider name BioLegend, product No. 304112), and an anti-CD49f-PE antibody (provider name BioLegend, product No. 313611). For each of the CD34⁺ fraction, CD34⁺CD38⁻CD133⁺ fraction, or CD34⁺CD38⁻CD45RA⁻CD49f⁺ fraction, which molecules have been reported as purification markers for human hematopoietic stem cells derived from human cord blood, the total cell count and the number of CD34⁺ cells included were counted by flow cytometry on day 7 after the start of culture, and the growth rate relative to that at the start of culture was determined. The results are shown in FIG. 31. As shown in FIG. 31, significant cell proliferation was observed in all cell fractions, but the CD34⁺CD38⁻CD133⁺ fraction and the CD34⁺CD38⁻CD45RA⁻CD49f⁺ fraction, especially the CD34⁺CD38⁻CD45RA⁻CD49f⁺ fraction exhibited marked cell growth.

### Example 3: Experiment for Long-Term Culture of Human Hematopoietic Stem Cells

Human hematopoietic stem cells were cultured in the above culture medium for human hematopoietic stem cells, which medium contained 20 µM PI3Ka and 0.1 µM TPOago instead of SCF and TPO. The total cell count and the CD34⁺ cell count were determined as above on days 7 and 14 after the start of culture. The results are shown in FIG. 32. As shown in FIG. 32, the total cell count increased with the number of days of incubation, while the CD34⁺ cell count decreased at day 14 compared to day 7.

When an optical microscope was used to observe cultured cells, giant cells were detected on day 14. To check the possibility that these giant cells were megakaryocytes or megakaryocyte progenitors, the cells were fractionated by flow cytometry using an anti-CD41a-FITC antibody (provider name BD Pharmingen, product No. 555466) and an anti-CD42b antibody (provider name BD Pharmingen, product No. 555473). As shown in FIG. 33, the results showed that the majority of cells at 14 days after the start of culture were CD41a⁺CD42b⁺ cells. However, as shown in FIG. 33, CD34⁺CD38⁻ cells were still proliferating under these conditions.

In addition, CD34⁺ cells were obtained from the culture at day 14 after the start of culture, and 100 cells were seeded into Methocult H4415 for colony assay. After 2 weeks, colonies were collected and cytospin specimens were prepared. The specimens were then subjected to Giemsa staining, and the colony types were determined under a microscope. The results are shown in FIG. 34. Regarding the colony types, G denotes "granulocytes"-, E denotes "erythroblasts"-, m denotes "macrophages"-, and M denotes "megakaryocytes"-containing colonies. As shown in FIG. 34, most of the cell colonies contained megakaryocytes.

### Example 4: Further Investigation of Conditions Suitable for Long-Term Culture of Human Hematopoietic Stem Cells

Long-term culture of human hematopoietic stem cells was attempted in the presence of compounds capable of growing human hematopoietic stem cells.

Prepared was the above culture medium for human hematopoietic stem cells, which medium contained 20 µM PI3Ka and 0.1 µM TPOago instead of SCF and TPO (PI3Ka20µM+TPOago0.1µM), or which medium contained SR-1 (500nM) and/or UM171 (35 nM) (+SR-1, +UM171, or +SR-1+UM171). In addition, purchased human bone marrow CD34⁺ cells (provider name: Lonza, product No. 2C-101) or CD34⁺ cells isolated using microbeads from given fresh cord blood as described above were used for culture experiments. First, 0.2 to 1.0 × 10⁵ cells per well of a 24-well plate were dispensed, and the CD34⁺ cells were cultured in the prepared medium. The total cell count and the CD34⁺ cell count were determined as above on day 14 after the start of culture, and the growth rate relative to that at the start of culture was determined. The results are shown in FIG. 35. As shown in FIG. 35, the total cell count and the CD34⁺ cell count were increased in the culture medium further containing UM171, and the growth rate of CD34⁺ cells was significantly larger in the medium containing UM171 than in the medium without UM171. In contrast, SR-1 caused cell death under these experimental conditions.

Further, the growth rate of the total cell count or the CD34⁺ cell count was determined by culturing the CD34⁺ cells under three conditions: condition 1: cultured for 14 days in the culture medium for human hematopoietic stem cells above, which medium contained 20 µM PI3Ka and 0.1 µM TPOago instead of SCF and TPO (P13Ka20µM+TPOago0.1µM), condition 2: at day 7 or later, cultured in a medium without butyzamide (Day7-no Buty), and condition 3: cultured for 14 days in a medium (PI3Ka20µM+TPOago0.1µM) further containing UM171 (+UM171). The CD41⁺ cell count was determined using a flow cytometer. The results are shown in FIG. 36. As shown in FIG. 36, the CD34⁺ cell count increased and the CD41⁺ cell count decreased under condition 2, in which the cells were cultured in the medium without butyzamide on day 7 or later after the start of culture, when compared to condition 1. As shown in FIG. 36, under condition 3, in which the cells were cultured in the medium further containing UM171, the growth rate of CD34⁺ cells was markedly increased and the CD41⁺ cell count was markedly decreased compared to condition 1 or 2. Thus, under serum-free medium conditions in the presence of PVA, PI3Ka and TPOago, UM171 was found to cause human hematopoietic stem cells to proliferate and to inhibit their differentiation into megakaryocyte progenitors and megakaryocytes (see FIG. 37).

### Example 5: Experiment for Transplantation of CD34⁺ Cells after Culture

Human CD34⁺ cells cultured for 7 days under each of conditions 1 to 3 were transplanted into irradiated NOG mice to check cell engraftment. Specifically, 1 × 10⁴ cultured human CD34+ cells were transplanted into each NOG mouse irradiated with 1.5 Gy of γ-rays. Twelve weeks after transplantation, peripheral blood of each NOG mouse was collected, and the cellular components in the peripheral blood were analyzed using a flow cytometer. The results are shown in FIG. 38. As shown in FIG. 38, in conditions 1 and 2, HSC (hematopoietic stem cell) engraftment rates increased from 14.9% and 11.1%, when pre-cultured CD34⁺ cells were engrafted, to 66.6% and 54.9%, respectively, when post-cultured CD34+ cells were engrafted. In condition 3, the HSC engraftment rate increased from 17%, when pre-cultured CD34⁺ cells were engrafted, to 67%, when post-cultured CD34⁺ cells were engrafted.

These results have revealed that the PI3K activator and the TPO receptor agonist can induce favorable proliferation of human hematopoietic stem cells under serum-free medium conditions in the absence of albumin and in the presence of PVA, and that the proliferated human CD34⁺ cells can maintain their HSC characteristics and the engraftment rate after transplantation into other individuals can be improved. In addition, human hematopoietic stem cells tend to proliferate as CD34⁺ cells, while some of them differentiate into megakaryocytes when cultured under the conditions for a long period, thereby producing megakaryocytes. The addition of UM171 to the cells increased the growth rate of CD34⁺ cells and inhibited their differentiation into megakaryocytes.

Next, human mononuclear cells were isolated from fresh human cord blood. Under conditions 1 and 3, the obtained cells (5 × 10⁵ cells) were cultured, and the total cell count, the CD34⁺ cell count, the percentage of CD34⁺ cells in the total cells, and the cell viability were each determined on day 7 and day 14 after the start of culture. The results are shown in FIG. 39. As shown in FIG. 39, under any of the conditions, the total cell count of mononuclear cells obtained from human cord blood decreased with incubation. On the other hand, under condition 3, the percentage of CD34⁺ cells in the total cells was markedly larger than under condition 1. The cell viability was also significantly higher in condition 3 than in condition 1. Further, the CD34⁺ cell count showed a marked increase in condition 3, while the cell count was maintained favorably in condition 1 whereas no increase in the cell count was observed.

### Example 6: Experiment of Culturing Human Hematopoietic Stem Cells in Presence of Polymer A

The above Examples have revealed that human hematopoietic stem cells, unlike mouse hematopoietic stem cells, require activation of the PI3K pathway, and that TPO can be replaced by butyzamide, a TPO receptor agonist. It has also been found that human hematopoietic stem cells can be cultured in the absence of UM171 when they are not cultured for a long period of time, but that differentiation into megakaryocyte lineages can be inhibited by culturing human hematopoietic stem cells in the presence of UM171 when they are cultured for a long period of time.

In experiments in this Example, human hematopoietic stem cells were cultured in the presence of polymer A, a PI3K activator, a TPO receptor agonist, and UM171, while PVA was replaced with polymer A in the above experiments. As a control experiment, human hematopoietic stem cells were cultured in the presence of PVA, a PI3K activator, a TPO receptor agonist, and UM171. PI3Ka was used as the PI3K activator and butyzamide as the TPO receptor agonist, and the concentrations were as in the above Examples.

The results are shown in FIG. 22. Then, as shown in FIG.22, human hematopoietic stem cells cultured in the presence of polymer A instead of PVA had a higher percentage of CD34-positive cells. In addition, the percentage of CD34-positive cells was higher in the presence of polymer A than in the presence of PVA. This has suggested that human hematopoietic stem cells proliferate well in the presence of polymer A and under conditions in which the PI3K pathway is activated (and in the absence of SCF). The results have also suggested that human hematopoietic stem cells can proliferate well even when TPO is replaced by a TPO receptor agonist under those conditions. It has also been found that human hematopoietic stem cells can proliferate well in the presence of UM171.

Further, the cell count changes were observed over time. In this experiment, human hematopoietic stem cells were cultured for 3 weeks while using a culture medium supplemented with a T cell expansion kit (Miltenyi Biotec) and human IL-2 (Peprotech). The results are shown in FIG. 23. As shown in FIG. 23, human hematopoietic stem cells proliferated equally in the presence of PVA or in the presence of polymer A.

### Example 7: To Culture Human CD3-Positive Cells

Human CD3-positive cells were stimulated with anti-CD28 and anti-CD3 antibodies using a T Cell Activation/Expansion Kit, human (Miltenyi Biotec) under regular conditions and cultured in an albumin-free and cytokine-free basal medium (IMDM medium containing 1% ITSX and 1% penicillin) containing 0.1% PVA or 0.1% Soluplus (trademark) for 6 weeks. The initial cell count was set to 1000 cells. The results are shown in FIG. 40.

As shown in FIG. 40, CD3-positive cells (mainly T cells) were demonstrated to proliferate well in any of the presence of Soluplus or the presence of PVA. This has demonstrated that an albumin-free culture medium can be used for the maintenance and proliferation of human T cells.

### Example 8: Differentiation of Human CD34-Positive Cells into Hematopoietic Cells

Human CD34-positive cells (hematopoietic stem cells) were cultured in an albumin-free basal medium containing PVA or Soluplus (trademark). The initial cell count was set to 1000 cells. Hematopoietic stem cells were differentiated into hematopoietic cells by adding a cytokine cocktail to the medium. The culturing was performed for 10 days. The results are shown in FIG. 41.

As shown in panel A of FIG. 41, the number of CD34-positive cells cultured in the culture medium increased favorably in any of the presence of Soluplus or the presence of PVA. As shown in panel B of FIG. 41, the cells in the culture medium as obtained by culturing in the presence of Soluplus contained megakaryocytes, erythroblasts, neutrophils, and macrophages. The results have demonstrated that almost all cells of blood cell lineages were obtained and that each of them proliferated, indicating that human blood cells can be expanded, maintained, and differentiated well in the presence of an additive such as PVA or Soluplus, even in an albumin-free environment.

### Example 9: To Culture Chronic Myelogenous Leukemia (CML) Cells

Bone marrow cell samples from CML (chronic myelogenous leukemia) patients were each cultured for one week under albumin-free and cytokine-free culture conditions. The culture medium used was a basal medium containing 0.1% PVA, 20 µM PI3Ka, and 0.1 µM TPOago. The culturing was performed in the presence or absence of an inhibitor (imatinib (IM)) of Bcr-Abl, the gene responsible for CML. The results are shown in FIG. 42.

As shown in FIG. 42, the results have demonstrated 0.5% cell proliferation in the absence of IM compared to the total cell count before culturing. In particular, more than 30-fold expansion/maintenance was observed in CD34-positive cells, a leukemia stem cell fraction, that is, more than 6-fold expansion/maintenance in absolute cell count. By contrast, no significant cell expansion was observed in the presence of IM, indicating that the cells proliferating in IM+ were CML leukemic stem cells.

## Claims

1. A method of culturing human cells, comprising
culturing human cells in a culture medium, wherein
the culture medium is a serum albumin-free medium and comprises an additive, the additive being selected from the group consisting of polyvinyl alcohol and modified polyalkylene glycol, and further comprises
(1) a phosphatidylinositol 3-kinase (PI3K) activator and at least one compound selected from the group consisting of thrombopoietin (TPO) and TPO receptor agonists;
(2) a TPO receptor agonist and at least one compound selected from the group consisting of stem cell factor (SCF) and PI3K activators; or
(3) a PI3K activator and a TPO receptor agonist, so that
the culturing allows the number of human hematopoietic stem cells or human blood cells to increase or be maintained.

2. The method according to claim 1, wherein the human cells are cells selected from the group consisting of human hematopoietic stem cells and human blood cells.

3. The method according to claim 1, wherein the human cells are blood cells other than hematopoietic stem cells.

4. The method according to claim 3, wherein the additive is polyvinyl alcohol.

5. The method according to claim 1 or 2, wherein the additive is modified polyalkylene glycol.

6. The method according to claim 5, wherein the modified polyalkylene glycol is polyalkylene glycol modified with a copolymer containing a polyvinylcaprolactam block and a polyvinylacetate block.

7. The method according to claim 6, wherein the modified polyalkylene glycol is polyethylene glycol modified with a copolymer containing a polyvinylcaprolactam block and a polyvinylacetate block.

8. A method of culturing human cells, comprising
culturing human cells in a culture medium, wherein
the culture medium comprises polyalkylene glycol modified with a copolymer containing a polyvinylcaprolactam block and a polyvinylacetate block.

9. The method according to claim 8, wherein the increased number of the human cells is obtained.

10. The method according to claim 8 or 9, wherein the human cells are human hematopoietic stem cells.

11. A culture medium composition for culturing human cells, comprising polyalkylene glycol modified with a copolymer containing a polyvinylcaprolactam block and a polyvinylacetate block.

12. A composition comprising human cells and polyalkylene glycol modified with a copolymer containing a polyvinylcaprolactam block and a polyvinylacetate block.

13. The composition according to claim 11 or 12, wherein the human cells are human hematopoietic stem cells.

14. Human cells obtained by the method according to any one of claims 8 to 10.
